(19) Europäisches Patentamt / European Patent Office / Office européen des brevets

(11) **EP 2 994 753 B1**

(12) **EUROPEAN PATENT SPECIFICATION**

(45) Date of publication and mention
of the grant of the patent:
**07.03.2018 Bulletin 2018/10**

(21) Application number: **13884053.3**

(22) Date of filing: **09.05.2013**

(51) Int Cl.:
*G01N 33/50* (2006.01)     *G06F 19/00* (2018.01)

(86) International application number:
**PCT/CN2013/075385**

(87) International publication number:
**WO 2014/179959 (13.11.2014 Gazette 2014/46)**

(54) **METHOD AND SYSTEM FOR ASSESSING HEALTH CONDITION**

VERFAHREN UND SYSTEM ZUR BEURTEILUNG DES GESUNDHEITSZUSTANDS

MÉTHODE ET SYSTÈME PERMETTANT D'ÉVALUER UN ÉTAT DE SANTÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**16.03.2016 Bulletin 2016/11**

(73) Proprietor: **The Procter & Gamble Company
Cincinnati, OH 45202 (US)**

(72) Inventors:
• **LI, Rui
  Beijing 101312 (CN)**
• **HUANG, Shi
  Qingdao
  Shandong 266101 (CN)**
• **HE, Tao
  Cincinnati, Ohio 45202 (US)**
• **LIU, Jiquan
  Singapore 138648 (SG)**
• **XU, Jian
  Qingdao
  Shandong 266101 (CN)**

(74) Representative: **Joos, Uta Susanne et al
P&G Manufacturing GmbH
Frankfurter Straße 145
61476 Kronberg (DE)**

(56) References cited:
**WO-A1-2011/079164     WO-A1-2012/100267
WO-A2-2013/062515**

• **BO LIU ET AL: "Deep Sequencing of the Oral
  Microbiome Reveals Signatures of Periodontal
  Disease", PLOS ONE, vol. 7, no. 6, 4 June 2012
  (2012-06-04), page e37919, XP055206046, DOI:
  10.1371/journal.pone.0037919**
• **RICARDO P. TELES ET AL: "Relationships
  between subgingival microbiota and GCF
  biomarkers in generalized aggressive
  periodontitis", JOURNAL OF CLINICAL
  PERIODONTOLOGY, vol. 37, no. 4, 1 April 2010
  (2010-04-01), pages 313-323, XP055206043, ISSN:
  0303-6979, DOI:
  10.1111/j.1600-051X.2010.01534.x**
• **MI ZHOU ET AL: "Investigation of the Effect of
  Type 2 Diabetes Mellitus on Subgingival Plaque
  Microbiota by High-Throughput 16S rDNA
  Pyrosequencing", PLOS ONE, vol. 8, no. 4, 1
  January 2013 (2013-01-01) , page e61516,
  XP055206057, ISSN: 1932-6203, DOI:
  10.1371/journal.pone.0061516**

EP 2 994 753 B1

**Description**

FIELD OF THE INVENTION

The present invention relates to a method of assessing whether a subject mammal has a target condition. The present invention also relates to a computer-aided system for assessing whether a subject mammal has a target condition. The present invention further relates to a computer-readable medium for assessing whether a subject mammal has a target condition.

BACKGROUND OF THE INVENTION

Health condition of a subject is customarily evaluated on the basis of a variety of symptoms. However, many of the symptoms used for health evaluation today, because of their subjective description and uncertain relationship to the disease state, are misleading.

Typically, an undesirable condition starts as an asymptomatic disorder which, if left untreated, progresses to a more serious condition. It can be difficult to detect such disorder in its early stage. Whilst doctors or other medical professionals are trained in disease detection, a proper examination is time consuming. Furthermore, even for a trained medical professional, quantification of the severity of the disorder is often difficult, and subjectivity in the assessment can lead to incorrect diagnosis. It is particularly difficult to assess the progression or remission of the disorder within an individual over time. As a consequence, when evaluating products or methods for treating such disorders, reliable clinical trials typically require large base sizes and may need to be run for several months in order to be able to detect differences between treatment products or methods, even though such differences may be clinically important. Other factors affecting such evaluations include high variability between test subjects, relative scarcity of suitable test subjects; and whilst the trial is being run, deviation from the desired protocol by individual test subjects, such as omission to use, or incorrect use of a treatment product or method. All of these make clinical trials very expensive to run, which in turn acts as a barrier in the development of effective treatment products or methods.

Much effort has been put into improving methods for assessing health conditions. A simple and well know method of assessing oral health condition is the use of a plaque disclosing product, which reveals the amount of bacterial plaque build-up on the teeth. Whilst the test is simple to perform, it focuses on those bacteria which are more harmful than others.

The oral cavity is a major site for microbial colonization. Oral microbial community varies among different individuals, different locations within the same oral cavity, or same location at different points in time. The differences in microbial community determine the oral microbial ecosystem, which is directly associated with oral health status and potentially overall systemic health status. Maintaining oral health is a key concern. Since many oral diseases are generally preventable and treatable, it is a modifiable risk factor for more serious systemic diseases. Early detection of warning signs that oral disease is or may be present is important to the prevention and treatment of diseases and maintenance of overall health.

Gingivitis, which involves inflammation of the soft tissues surrounding the teeth, is one of the most prevalent infections and the most common oral disease in humans. As a worldwide health concern, it affects most children and adolescents. The disease is believed to be a result from build-up of bacterial plaque and ensuing interactions between the plaque microbiota and host tissues. Although no apical migration of the junctional epithelium occurs, these tissues become erythematous and bleed upon probing. Moreover, chronic gingivitis can progress to periodontitis, which is an irreversible periodontal infection characterized by alveolar bone loss, attachment loss, formation of periodontal pockets, and eventually tooth loss. Therefore, preventive measures against gingivitis, and improved tools for prognosis and early diagnosis thereof, are of particular clinical importance.

Several factors have hindered investigation of the etiology of gingivitis, which remains poorly understood. In natural human populations, gingivitis symptoms can be reversible and volatile, as numerous internal or external factors, including oral hygiene practices (personal or professional), impairment of immune system, injury, diet and oral state, may all potentially affect disease development, thereby confounding disease monitoring. Moreover, clinical diagnosis of gingivitis is based on individual observations and judgment by human examiners. Consequently, the results can be difficult to compare between different patients and different examiners. Furthermore, despite the complexity of oral microbial communities and the suspected polymicrobial nature of chronic oral infections, population-wide surveys of gingivitis-associated microbiota have been limited to only a few culturable bacteria (e.g. the "red complex" including *Porphyromonas gingivalis, Tannerella forsythia,* and *Treponema denticola*), which provide insufficient data points for a thorough analysis of various microbes that may potentially cause gingivitis. Bo Liu et al., Plos One, vol. 7, no. 6, June 2012 p. e37919 analyzed the oral microbiome of healthy and diseased subgingival plaque by 16S rRNA sequencing. Ricardo P. Teles et al., Journal of Clinical Periodontology, vol. 37, no. 4, 1 April 2010, p. 313-123 discloses a method of assessing samples with aggressive periodontitis, by analyzing 40 bacterial species and the concentration of cytokines in subgingival plaque. Mi Zhou et al., Plos One, vol. 8, no. 4, 1 January 2013, p. e 61516 analyzed the microbiome of periodontal disease in patients with or without diabetes. WO2012/100267A1 generally discloses a method to asses if a patient has a target condition by defining groups of biomarkers that are increased or decreased in patients with the condition compared to normal samples.

Accordingly, there continues to be a need for improved diagnostic capabilities for assessing the health condition of a subject by analyzing the microbiome of the oral cavity. There continues to be a need for an objective, reproducible and sensitive measure of a subject's health condition. There continues to be a need for early detection of disease well before symptoms appear so that early intervention and preventive measures can be taken. There continues to be a need for accurate determination of a subject's susceptibility to a disease so as to better prevent and control development of undesirable conditions and diseases.

SUMMARY OF THE INVENTION

To address these challenges and/or needs, the present invention takes a properly balanced oral environment into consideration for assessing the health condition, specifically in terms of a balance in the oral microbial community.
In one aspect, the present invention relates to a method of assessing whether a subject mammal has a target condition, comprising the steps:

a) defining the target condition, wherein the target condition is selected from the group consisting of a disease, severity of a disease, sensitivity to a disease, and combinations thereof and the disease is a microbe-related disease;
b) defining a first group of biomarkers each having a higher abundance in oral cavities of a set of test mammals with said target condition compared to oral cavities of a set of test mammals without said target condition, wherein the first group of biomarkers are bacterial genera selected from the group consisting of *Leptotrichia, Prevotella, Fusobacterium,* TM7, *Porphyromonas, Tannerella, Selenomonas, Lachnospiraceae, Comamonadaceae, Peptococcus, Aggregatibacter, Catonella, Treponema,* SRI, *Campylobacter, Eubacterium, Peptostreptococcus, Bacteroidaceae, Solobacterium, Johnsonella, Oribacterium, Veillonellaceae,* and combinations thereof;
c) defining a second group of biomarkers each having a lower abundance in the oral cavities of the set of test mammals with said target condition compared to the oral cavities of the set of test mammals without said target condition, wherein the second group of biomarkers are bacterial genera selected from the group consisting of *Streptococcus, Rothia, Actinomyces, Haemophilus, Lautropia,* and combinations thereof.;
d) formulating a linear function of the abundances of the first group of biomarkers and the abundances of the second group of biomarkers that is useful for assessing whether the subject mammal has the target condition comprising a formula:

$$f(Ai, Aj) = b\left(\frac{\sum_{i \in N} Ai}{N} - \frac{\sum_{j \in M} Aj}{M}\right)$$

where $N$ is a total number of the biomarkers in the first group, $M$ is a total number of the biomarkers in the second group, $Ai$ is an abundance of each biomarker $i$ in the first group, $Aj$ is an abundance of each *biomarker j* in the second group, $\sum_{i \in N} Ai$ is a sum of $Ai$ over all biomarkers $i$ in the first group, $\sum_{j \in M} Aj$ is a sum of $Aj$ over all biomarkers $j$ in the second group, and b is a constant;
e) obtaining a sample from an oral cavity of the subject mammal, wherein the obtained sample is capable of containing the first group of biomarkers and the second group of biomarkers;
f) measuring abundances of the first group of biomarkers in the obtained sample from the subject mammal;
g) measuring abundances of the second group of biomarkers in the obtained sample from the subject mammal; and
h) inputting the measured abundances of the first group and the second group of biomarkers into the formulated function to assess whether the subject mammal has the target condition.

In another aspect, the present invention relates to a computer-aided system for assessing whether a subject mammal has a target condition, comprising:

a) a sampling section configured for sampling an oral cavity sample of the subject mammal, wherein the sampled oral cavity sample is capable of containing:

i) a first group of biomarkers each having a higher abundance in oral cavities of a set of test mammals with said target condition compared to oral cavities of a set of test mammals without said target condition, wherein the first group of biomarkers are bacterial genera selected from the group consisting of *Leptotrichia, Prevotella,*

*Fusobacterium,* TM7, *Porphyromonas, Tannerella, Selenomonas, Lachnospiraceae, Comamonadaceae, Peptococcus, Aggregatibacter, Catonella, Treponema,* SRI, *Campylobacter, Eubacterium, Peptostreptococcus, Bacteroidaceae, Solobacterium, Johnsonella, Oribacterium, Veillonellaceae,* and combinations thereof; and
ii) a second group of biomarkers each having a lower abundance in the oral cavities of the set of test mammals with said target condition compared to the oral cavities of the set of test mammals without said target condition, wherein the second group of biomarkers are bacterial genera selected from the group consisting of *Streptococcus, Rothia, Actinomyces, Haemophilus, Lautropia,* and combinations thereof.;

b) a measuring section in communication with the sampling section, wherein said measuring section is configured for measuring the sampled oral cavity sample to obtain abundances of the first group and the second group of biomarkers in the sampled oral cavity sample; and
c) a computing section in communication with the measuring section, wherein said computing section stores a linear function of abundances of the first group of biomarkers and abundances of the second group of biomarkers that is useful for assessing whether the subject mammal has the target condition comprising a formula:

$$f(Ai, Aj) = \mathrm{b}\left(\frac{\sum_{i \in N} Ai}{N} - \frac{\sum_{j \in M} Aj}{M}\right)$$

where $N$ is a total number of the biomarkers in the first group, $M$ is a total number of the biomarkers in the second group, $Ai$ is an abundance of each biomarker $i$ in the first group, $Aj$ is an abundance of each *biomarker $j$* in the second group, $\sum_{i \in N} Ai$ is a sum of $Ai$ over all biomarkers i in the first group, $\sum_{j \in M} Aj$ is a sum of $Aj$ over all biomarkers $j$ in the second group, and b is a constan, and wherein the computing section is configured for applying the function to the obtained abundances of the first group and the second group of biomarkers in the sampled oral cavity sample to assess whether the subject mammal has the target condition.

In a further aspect, the present invention relates to a computer-readable medium for assessing whether a subject mammal has a target condition, comprising:

a) a memory storing a function of abundances of a first group of biomarkers and abundances of a second group of biomarkers that is useful for assessing whether the subject mammal has the target condition,, wherein the first group of biomarkers are bacterial genera selected from the group consisting of *Leptotrichia, Prevotella, Fusobacterium,* TM7, *Porphyromonas, Tannerella, Selenomonas, Lachnospiraceae, Comamonadaceae*, *Peptococcus, Aggregatibacter, Catonella, Treponema,* SRI, *Campylobacter, Eubacterium, Peptostreptococcus, Bacteroidaceae, Solo bacterium, Johnsonella, Oribacterium, Veillonellaceae,* and combinations thereof; and wherein the second group of biomarkers are bacterial genera selected from the group consisting of *Streptococcus, Rothia, Actinomyces, Haemophilus*, *Lautropia,* and combinations thereof wherein
each of the first group of biomarkers has a higher abundance in oral cavities of a set of test mammals with said target condition compared to oral cavities of a set of test mammals without said target condition, and
each of the second group of biomarkers has a lower abundance in the oral cavities of the set of test mammals with said target condition compared to the oral cavities of the set of test mammals without said target condition; and
b) a computer code comprising instructions for applying the linear function to a data set obtained from the subject mammal, wherein the data set comprises abundances of the first group and the second group of biomarkers in an oral cavity sample of the subject mammal, assessing whether the subject mammal has the target condition.

By the method and system described herein, the present invention provides an objective, reproducible and sensitive measure of a health condition, especially prior to or immediately upon appearance of symptoms of a disease development. Further, the present invention provides a relatively convenient means of assessing health condition and/or evaluating treatment products and interventions (e.g., compared to clinical trials).
These and other features, aspects, and advantages of the present invention will become evident to those skilled in the art from the detailed description which follows.

BRIEF DESCRIPTION OF THE DRAWINGS

While the specification concludes with claims particularly defining and distinctly claiming the invention, it is believed that the invention will be better understood from the following description of the accompanying figures. In the accompanying figures,

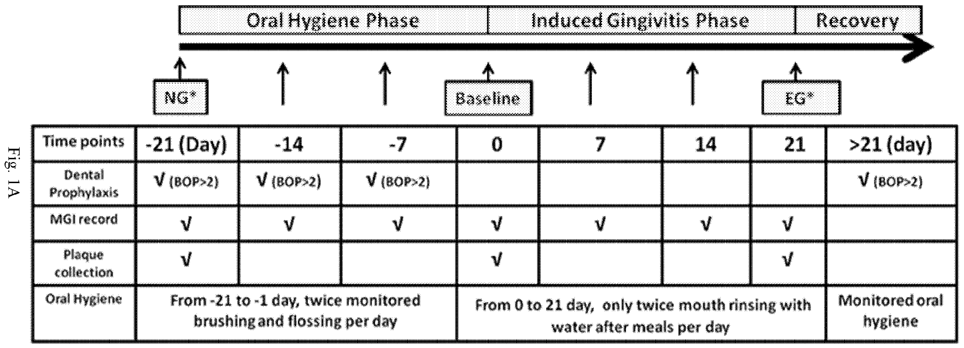

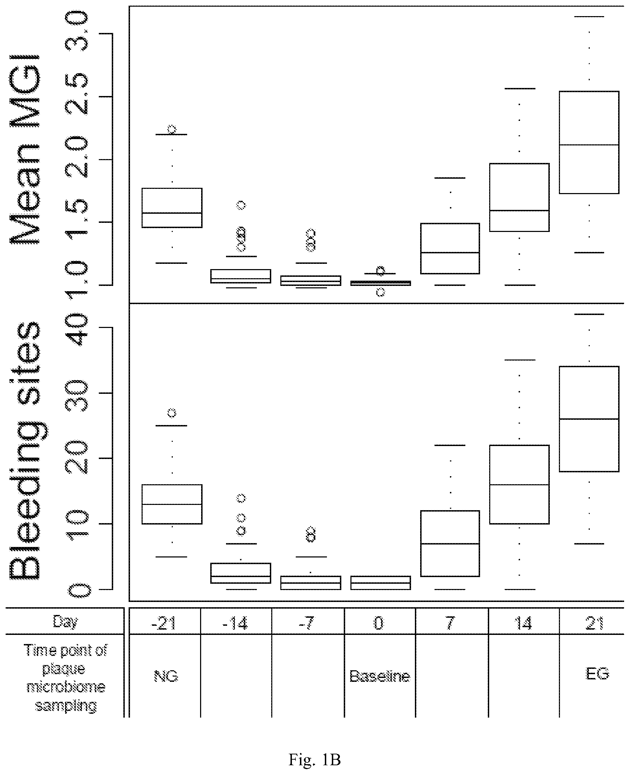

Fig. 1A illustrates a design of longitudinal study simulating gingivitis development in human population according to a specific embodiment of the present invention. Fig. 1B shows values of certain clinical parameters for 50 subjects throughout the study at different time points.

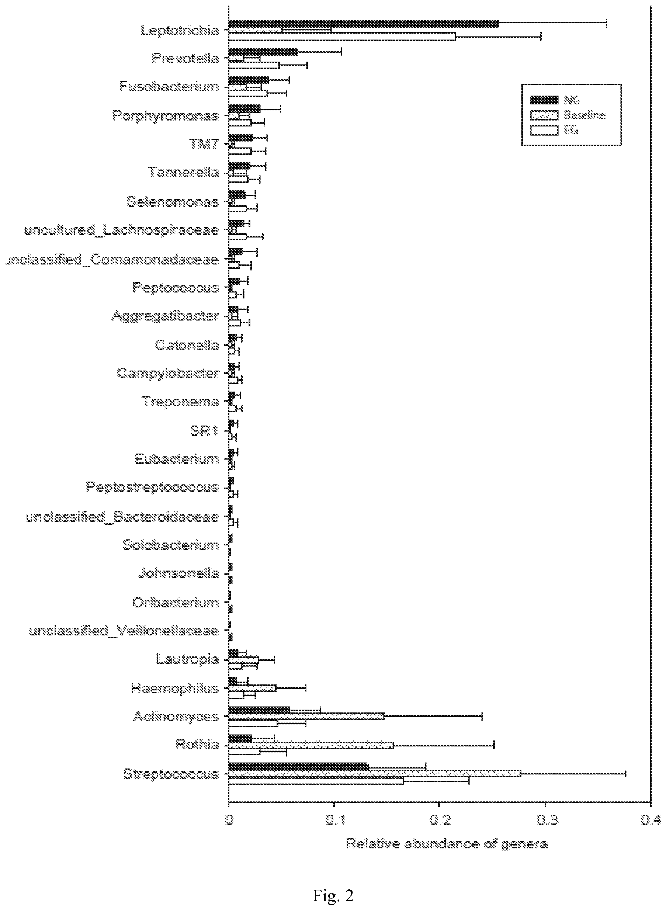

Fig. 2 shows the abundances of 27 genus-level bacterial biomarkers that distinguish between a healthy state and gingivital state(s) (including both naturally occurring gingivitis state and experimentally induced gingivitis state) in a set of test subjects, according to a specific embodiment of the present invention.

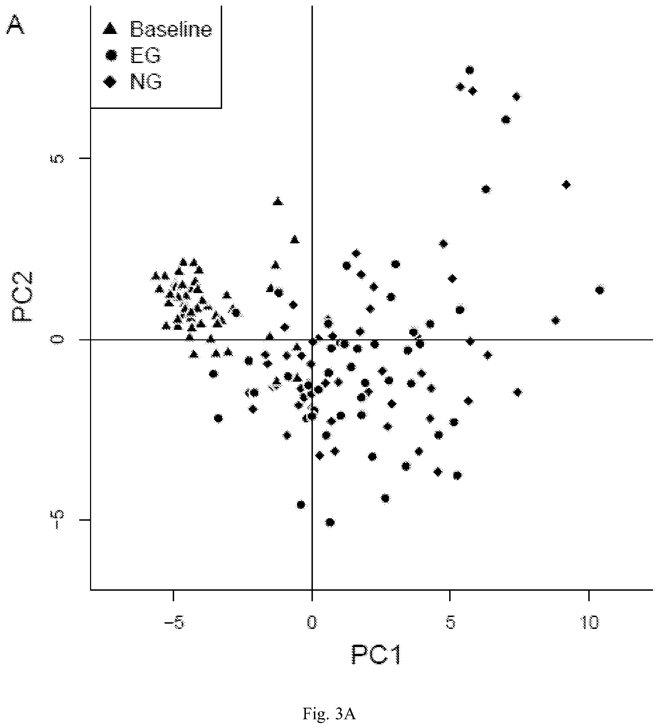

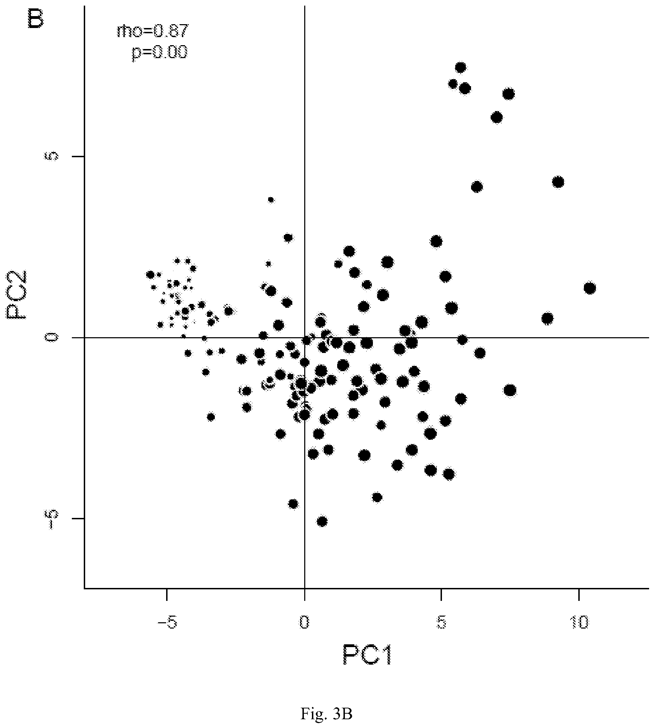

Figs. 3A and 3B show plots of principal components 1 and 2 (PC1 and PC2) from a principal component analysis (PCA) of genus-level bacteria data measured for 150 oral cavity samples collected from 50 subjects at three different stages, i.e., a naturally occurring gingivital stage ("NG"), a baseline stage ("Baseline"), and an experimentally induced gingivital stage ("EG"), according to a specific embodiment of the present invention.

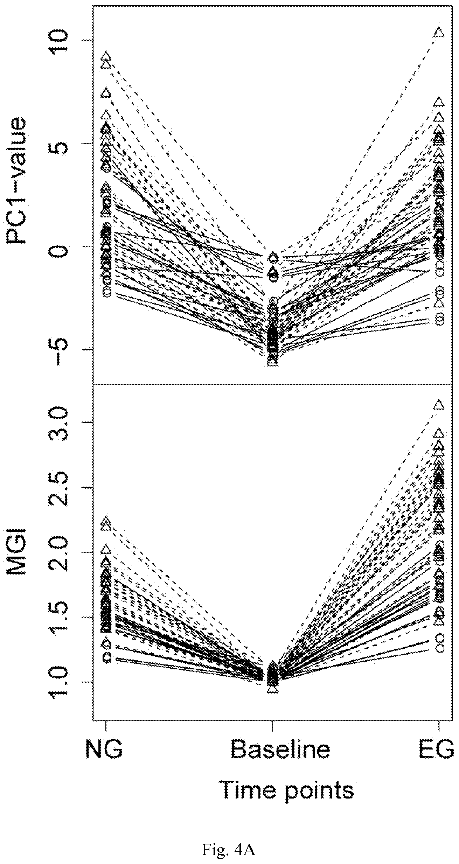

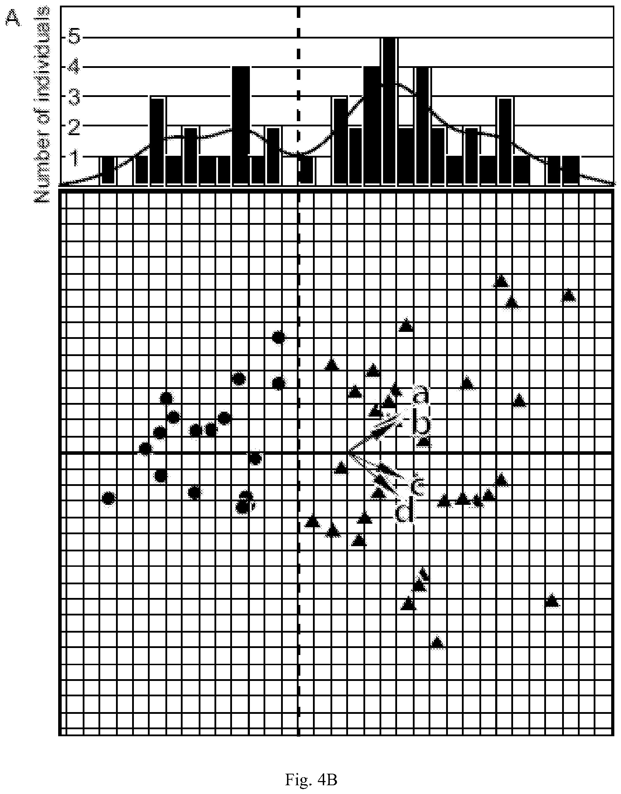

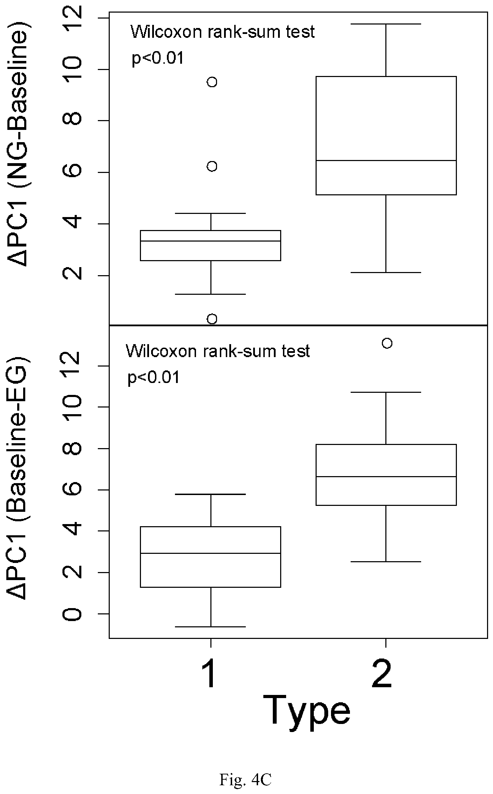

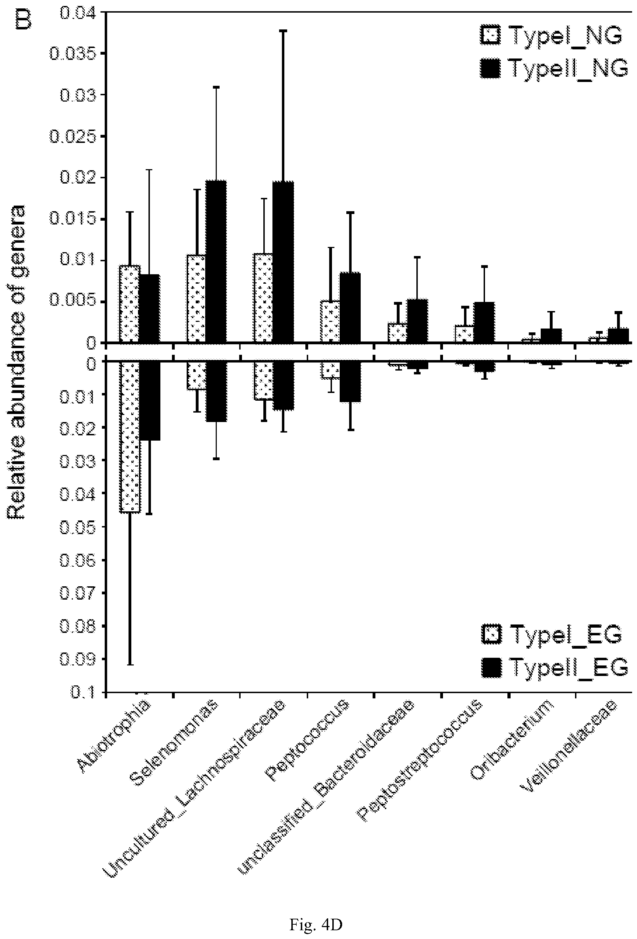

Figs. 4A, 4B, 4C, and 4D show the identification of two types of hosts with distinct sensitivity to gingivitis according to a specific embodiment of the present invention. Fig. 4A shows patterns of microbiota structural (i.e. PC1-values) change and Mazza Gingival Index change along RPM. Fig. 4B shows distribution of the 50 subjects along principal components 1 and 2 (PC1 and PC2) of the PCA, wherein the vertical dash line divides the 50 subjects into Type-I and Type-II hosts. Fig. 4C shows difference in gingivitis sensitivity between Type-I and Type-II hosts. Fig. 4D shows the abundances of 8 genus-level bacterial biomarkers that distinguish between Type-I and Type-II hosts.

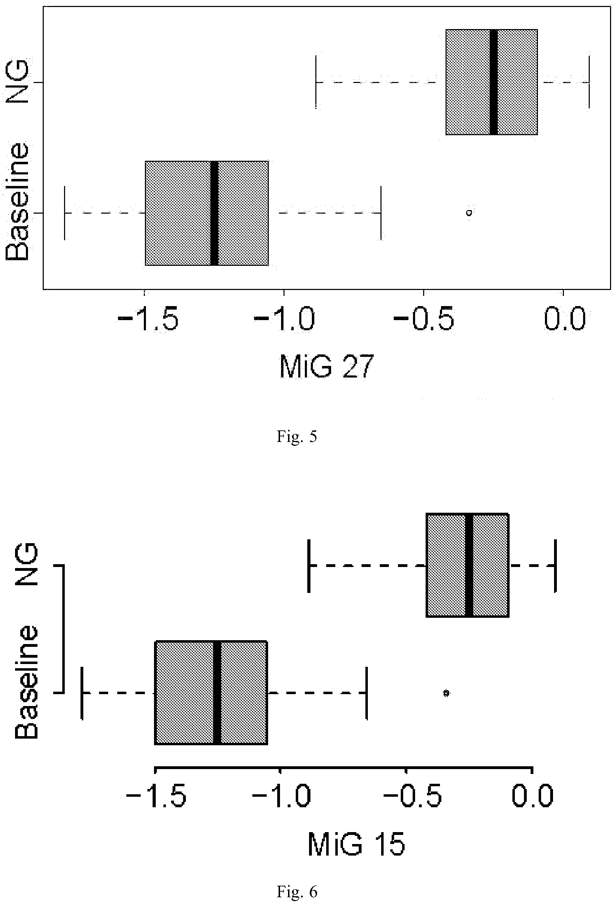

Fig. 5 shows a trial classification based on the presence of gingivitis using a microbial index of gingivitis, MiG27, which is calculated from a function based on abundances of 27 biomarkers defined according to a specific embodiment of the present invention.

Fig. 6 shows a trial classification based on the severity of gingivitis using a microbial index of gingivitis, MiG15, which is calculated from a function based on abundances of 15 biomarkers defined according to another specific embodiment of the present invention.

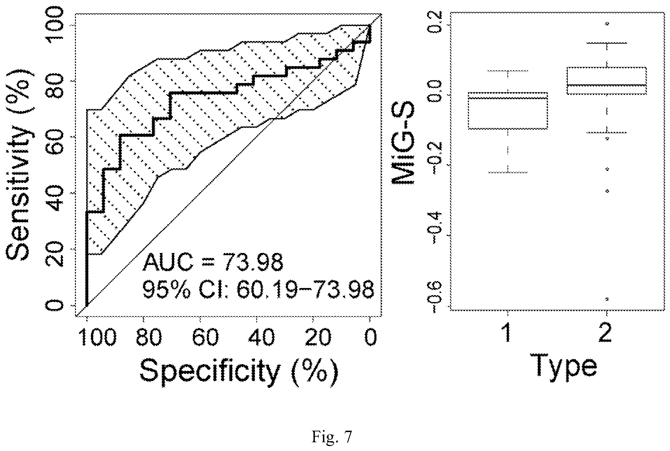

Fig. 7 shows a trial classification based on the sensitivity to gingivitis using a microbial index of gingivitis, MiG-S, which is calculated from a function based on abundances of 8 biomarkers defined according to a further specific embodiment of the present invention. The accuracy of MiG-S is measured by the area under the ROC (receiver operating characteristic) curve of plaque-microbiota-based (i.e. MiG-S-based) gingivitis-sensitive host-type classification as shown in the left diagram.

DETAILED DESCRIPTION OF THE INVENTION

## Definitions

As used herein, the term "mammal" refers to any of various warm-blooded vertebrate animals of the class Mammalia, including humansIn the context herein, the mammal can also be called "subject" or "host".

As used herein, the term "a set of mammals" means a number of mammals gathered together into a group for the purpose of study. The number in the set can be any countable number no less than 1. Depending on the purpose accuracy requirement of a specific study, the number of mammals in the set can be up to 1000, 10000 or even larger.

As used herein, the terms "microbial community", "microbiota", "microflora", "microbial flora" and "flora" are used interchangeably herein and refer to a population of diverse microorganisms that typically inhabits the mammal, specifically an organ (e.g., skin, digestive tract) or an orifice (e.g, mouth) of the mammal. The term "microorganism" means an organism of microscopic or submicroscopic size, especially a bacterium or protozoan, more preferably bacterium.

As used herein, the term "microbe-related disease" includes an illness in the mammal caused or influenced or associated by a microorganism.

As used herein, the term "biomarker" includes indicators or markers present or absent in the biological system, site or sample that indicate occurrence of a biological process or event.

As used herein, the terms "sample" or "biological sample" is a biological material isolated from a subject for analysis according to the present methods, such as saliva, gingival crevicular fluid (GCF), supragingival plaque, subgingival plaque, breath or exhaled air, oral lavage, tongue scrapings, swabs or biopsies from oral tissue and serum. A sample is ideally capable of containing a microbial community.

As used herein, the term "statistical significance" is a mathematical tool that is used to determine whether the outcome of an experiment is the result of a relationship between specific factor(s) or merely the result of chance. Statistical significance is used to reject or accept what is called the null hypothesis. A hypothesis is an explanation that a researcher is trying to prove. The null hypothesis typically holds that the factor(s) at which a researcher is looking have no effect on differences in the data or that there is no connection between the factors. Statistical significance is usually written, for example, as $t=0.02$, $p<0.05$. Here, "$t$" stands for the test score and "$p<0.05$" means that the probability of an event occurring by chance is less than 5 percent. These numbers would cause the null hypothesis to be rejected.

As used herein, with reference to a disease or condition, the term "sensitivity" and its adjective form "sensitive" can be used interchangeably with "susceptibility" and its adjective form "susceptible" to mean the likelihood of suffering from a disease or condition when exposed to a noxious stimulus or pathogen.

As used herein, the articles including "a" and "an" when used in a claim, are understood to mean one or more of what is claimed or described.

As used herein, the terms "comprise", "comprises", "comprising", "include", "includes", "including", "contain", "contains", and "containing" are meant to be non-limiting, i.e., other steps and other sections which do not affect the end of result can be added. The above terms encompass the terms "consisting of" and "consisting essentially of".

### Target Condition

The present invention provides a method of assessing whether a subject mammal has a target condition. The target condition can be any condition which is used to describe a mammal's health status, including but not limited to presence of a disease, severity of a disease, sensitivity to a disease, and combinations thereof.

According to a specific embodiment, the disease is a micro-related disease, preferably selected from the group consisting of gingivitis, periodontitis, dental caries, halitosis, oral ulcer, premature birth, low birth weight, diabetes, respiratory disease, heart disease, stroke, bacteremia, whole body health, and combinations thereof.

### Sample Collection & Storage

Depending on the specific condition, the sample from an oral cavity, preferably in the form of a biofilm on the surfaces of the teeth, prostheses (when present), gums, and tongue, can be selected from the group consisting of a salivary sample, a plaque sample, a tongue dorsum sample, a tongue coating sample, a mucous membrane sample, and combinations thereof. The plaque sample can be from various locations. For example, the plaque sample can be selected from the group consisting of a supragingival plaque sample, a subgingival plaque sample, a tooth plaque sample, and combinations thereof. The selection of the sample may be critical to the accuracy of assessing the target condition. For example, plaque microbiota is believed to be more sensitive to gingivitis than salivary microbiota. Therefore, in the case of gingivitis, the sample is preferably a plaque sample.

The samples, once collected, can be used in subsequent steps immediately. Alternatively, the samples can be put in a freezer for later use. In some cases, the newly collected samples are immediately deep frozen, typically below -20°C, preferably below -50°C, more preferably below -70°C, and most preferably below -90°C. The samples remain frozen until preparation for analysis.

### Biomarkers

The potential of microbiota for tracking and diagnosing a mammal's condition (diseases, diets, etc.) is dependent on, and limited by, the degree of heterogeneity in the link between the microbiota and the condition at the population level. In the gut, the variation of microbiota structure between subjects appears to dominate variation among conditions (e.g. lean or obese, or on a normal or high-fat diet). However, the present inventors surprisingly found that the opposite appears to be true for oral microbiota. That is, it is surprisingly found that the differences between healthy and diseased oral microbiota within a subject are larger than inter-subject differences. This suggests that the oral microbiota might offer certain advantages as biomarkers for oral, and perhaps even systemic, diseases.

Oral microbial community comprises an extremely diverse microflora, some of which are potentially harmful or "bad"; and some of which are not harmful or even beneficial, as to beconsidered as "good" bacteria, in part because they serve to prevent proliferation of other more harmful organisms. Thus, achieving a healthy oral status does not necessarily require eradicating all bacteria, but it is important to maintain a certain balance between the "good" bacteria and the "bad" bacteria. For example, "good" bacteria typically include the genus *Lactobacillus.* The most prevalent strains in healthy persons include *Lactobacillus gasseri* and *Lactobacillus fermentum* and the strongest antimicrobial activity is associated with strains including *L. paracasei, L. plantarum, L. rhamnosus, and L. salivarius*. "Bad" or harmful bacteria include, for example, *Streptococcus mutans, Tannerella forsythia, Porphyromonas gingivalis,* and *F. nucleatum.*

Many studies have demonstrated a shift in the microbial community from prevalent "good" biomarkers to "bad" biomarkers when a disease occurs. For example, a shift is reported in the microbial community from a predominately gram-positive facultative aerobic to a predominately gram-negative anaerobic flora correlated with the formation of foul odors from incubated saliva (T.F. McNamara, et al., Oral Surg Oral Med. Oral Pathol. (1972), 34(1):41-8; J. Tonzetich, J. Periodontol. (1977), 48(1):13-20). In the oral cavity, the most common consequences of imbalance of microbiota are dental caries, halitosis and gingivitis/periodontitis. The status of gingivitis/periodontitis can be predicted by a characteristic microbial shift from the early prevalence of Gram-positive facultative microorganisms (e.g., *Streptococcus* spp., *Streptococcus saginus, Actinomyces* spp., and *A. naeslundii*) to the later prevalence of Gram-negative anaerobic microorganisms (e.g., *Porphyromonas gingivalis* and *P. endodontalis, Tannerella forsythia, Aggregatibacter actinomycetemcomitans, Treponema denticola* and *T. socranskii, Prevotella intermedia Fusobacterium nucleatum, Eikenella corrodens,*

*Campylobacter rectus and C. gracilis,* and *Veillonella parvula*); and the status of dental caries can be predicted by a shift from non-aciduric bacteria (e.g., *Streptococcus saginus* and *Actinomyces* spp.) to aciduric bacteria (e.g., *Streptococcus mutans, Streptococcus sobrinus, Lactobacillus* spp., and *Bifidobacterium* spp.).

According to the present invention, a first group of biomarkers and a second group of biomarkers are defined to include "bad" biomarkers and "good" biomarkers, respectively. Including but not limited to those disclosed as "bad" and "good" biomarkers in the prior art, it is believed that, the first group of biomarkers each has a higher abundance in oral cavities of a set of test mammals with the target condition compared to oral cavities of a set of test mammals without the target condition, and the second group of biomarkers each has a lower abundance in the oral cavities of the set of test mammals with said target condition compared to the oral cavities of the set of test mammals without said target condition. Preferably, the set of test mammals without the target condition is a control set of test mammals.

According to a specific embodiment, the biomarkers are each independently selected from the group consisting of taxonomic categories of a bacterium, functional categories of a microbe, and combinations thereof. More specifically and preferably, the biomarkers are each independently selected from the group consisting of a bacterial phylum, a bacterial class, a bacterial family, a bacterial order, a bacterial genus, a bacterial species, a functional gene of a microbe, a gene ortholog group of a microbe, a motif of peptide or protein of a microbe, a conserved peptide or protein domain of a microbe, a none-coding nucleotide sequence of a microbe, and combinations thereof, preferably a bacterial genus.

Many techniques can be used to measure abundance of a biomarker in the sample. On one hand, by selecting a particular population of microorganisms, culture-based methods can be used to investigate the microbial ecology of natural and anthropogenically impacted environments. Standard culture techniques to characterize microbial ecology involve isolation and characterization of microorganisms using commercial growth media such as Luria-Bertani medium, Nutrient Agar, and Tryptic Soy Agar. The major limitation of culture-based techniques is that >99% of the microorganisms in any environment observed through a microscope are not cultivable by standard culturing techniques. On the other hand, with recent advances in genomics and sequencing technologies, a variety of culture-independent molecular methods based on direct isolation and analysis of nucleic acids, proteins, and lipids from samples have been discovered and revealed structural and functional information about microbial communities. Molecular approaches such as genetic fingerprinting, metagenomics, metaproteomics, metatranscriptomics, and proteogenomics are vital for discovering and characterizing the vast microbial diversity and understanding their interactions with biotic and abiotic environmental factors.

According to a specific embodiment, the abundance of a biomarker in the sample is measured by one or more methods selected from the group consisting of 16S rRNA(RiboNucleic Acid) analysis, genetic fingerprinting, clone library method, denaturing- or temperature-gradient gel electrophoresis, random amplified polymorphic DNA(DeoxyriboNucleic Acid), DNA amplification fingerprinting, amplified ribosomal DNA restriction analysis, DNA microarrays, fluorescence *in situ* hybridization, DNA-DNA hybridization, metagenomics, metaproteomics, metatranscriptomics, proteogenomics, Luria-Bertani medium isolation technique, Nutrient Agar isolation technique, Tryptic Soy Agar isolation technique, and any combination thereof. Molecular analyses of microbial communities have revealed that the cultivable fraction represents <1% of the total number of prokaryotic species present in any given sample. Preferably, a method selecting from the group consisting of 16S rRNA analysis, metagenomics, and combination thereof is used in the present invention to measure the sample, obtaining abundances of one or more biomarkers. Most preferably, 16S rRNA analysis is used to study the microbial communities of the samples.

## Method of Assessing a Condition

One aspect of the invention provides for a method of assessing whether a subject mammal has a target condition comprises the steps:

a) defining the target condition;
b) defining a first group of biomarkers each having a higher abundance in oral cavities of a set of test mammals with said target condition compared to oral cavities of a set of test mammals without said target condition;
c) defining a second group of biomarkers each having a lower abundance in the oral cavities of the set of test mammals with said target condition compared to the oral cavities of the set of test mammals without said target condition;
d) formulating a function of the abundances of the first group of biomarkers and the abundances of the second group of biomarkers that is useful for assessing whether the subject mammal has the target condition;
e) obtaining a sample from an oral cavity of the subject mammal, wherein the obtained sample is capable of containing the first group of biomarkers and the second group of biomarkers;
f) measuring abundances of the first group of biomarkers in the obtained sample from the subject mammal;
g) measuring abundances of the second group of biomarkers in the obtained sample from the subject mammal; and
h) inputting the measured abundances of the first group and the second group of biomarkers into the formulated function to assess whether the subject mammal has the target condition.

According to a specific embodiment, the function of the abundances of the first group of biomarkers and the abundances of the second group of biomarkers is selected from the group consisting of a linear function, a quadratic function, a cubic function, a quartic function, a quintic function, a sextic function, a rational function, and combinations thereof.

According to a further specific embodiment, the function of the abundances of the first group of biomarkers and the abundances of the second group of biomarkers is a linear function, preferably comprising a formula:

$$f(Ai, Aj) = b\left(\frac{\sum_{i \in N} Ai}{N} - \frac{\sum_{j \in M} Aj}{M}\right)$$

where $N$ is a total number of the biomarkers in the first group, $M$ is a total number of the biomarkers in the second group, $Ai$ is an abundance of each biomarker $i$ in the first group, $Aj$ is an abundance of each biomarker $j$ in the second group, $\sum_{i \in N} Ai$ is a sum of $Ai$ over all biomarkers $i$ in the first group, $\sum_{j \in M} Aj$ is a sum of $Aj$ over all biomarkers $j$ in the second group, and b is a constant which, in a particular embodiment, is selected from the range from 1 to 10000, preferably from 5 to 1000, more preferably from 6 to 100, and most preferably from 10 to 50.

According to a specific embodiment, the target condition is selected from the group consisting of gingivitis, severity of gingivitis, sensitivity to gingivitis, and combinations thereof.

According to a specific embodiment, the first group of biomarkers is bacterial genera selected from the group consisting of *Leptotrichia, Prevotella, Fusobacterium,* TM7, *Porphyromonas, Tannerella, Selenomonas, Lachnospiraceae, Comamonadaceae, Peptococcus, Aggregatibacter, Catonella, Treponema,* SRI, *Campylobacter, Eubacterium, Peptostreptococcus, Bacteroidaceae, Solobacterium, Johnsonella, Oribacterium, Veillonellaceae,* and combinations thereof; and the second group of biomarkers are bacterial genera selected from the group consisting of *Streptococcus, Rothia, Actinomyces, Haemophilus, Lautropia,* and combinations thereof. These biomarkers are especially useful for assessing whether a subject mammal has gingivitis, when the function of the abundances of the first group of biomarkers and the abundances of the second group of biomarkers is:

$$f(Ai, Aj) = b\left(\frac{\sum_{i \in 22} Ai}{22} - \frac{\sum_{j \in 5} Aj}{5}\right)$$

where $Ai$ is an abundance of each biomarker $i$ in the first group, $Aj$ is an abundance of each biomarker $j$ in the second group, $\sum_{i \in 22} Ai$ is a sum of $Ai$ over all biomarkers $i$ in the first group, $\sum_{j \in 5} Aj$ is a sum of $Aj$ over all biomarkers $j$ in the second group, and b is a constant, preferably selected from the range from 1, 3, 5, 8, or 10 to 20, 50, 100, 500, or 10000, alternatively selected from the range from 1 to 9, 15 to 200, 30 to 600, 800 to 1500, or combinations thereof.

According to a specific embodiment, the first group of biomarkers is bacterial genera selected from the group consisting of *Prevotella, Leptotrichia, Fusobacterium, Selenomonas, Lachnospiraceae,* TM7, *Tannerella, Peptococcus, Peptostreptococcus, Catonella, Treponema, Solobacterium, Bacteroidaceae,* and combinations thereof; and the second group of biomarkers are bacterial genera selected from the group consisting of *Rothia, Haemophilus,* and combination thereof. These biomarkers are especially useful for assessing whether a subject mammal has severe or non-severe gingivitis, when the function of the abundances of the first group of biomarkers and the abundances of the second group of biomarkers is:

$$f(Ai, Aj) = b\left(\frac{\sum_{i \in 13} Ai}{13} - \frac{\sum_{j \in 2} Aj}{2}\right)$$

where $Ai$ is an abundance of each biomarker $i$ in the first group, $Aj$ is an abundance of each biomarker $j$ in the second group, $\sum_{i \in 13} Ai$ is a sum of $Ai$ over all biomarkers $i$ in the first group, $\sum_{j \in 2} Aj$ is a sum of $Aj$ over all biomarkers $j$ in the second group, and b is a constant, preferably selected from the range from 1, 3, 5, 8, or 10 to 20, 50, 100, 500, or 10000, alternatively selected from the range from 1 to 9, 15 to 200, 30 to 600, 800 to 1500, or combinations thereof.

According to a specific embodiment, the first group of biomarkers is bacterial genera selected from the group consisting of *Selenomonas, Lachnospiraceae, Peptococcus, Bacteroidaceae, Peptostreptococcus, Oribacterium, Veillonellaceae* and combinations thereof; and the second group of biomarkers is a bacterial genus of *Abiotrophia.* These biomarkers are especially useful for assessing whether a subject mammal is sensitive or non-sensitive to gingivitis, when the function of the abundances of the first group of biomarkers and the abundances of the second group of biomarkers is:

$$f(Ai, Aj) = b\left(\frac{\sum_{i \in 7} Ai}{7} - \frac{\sum_{j \in 1} Aj}{1}\right)$$

where $Ai$ is an abundance of each biomarker $i$ in the first group, $Aj$ is an abundance of each biomarker $j$ in the second group, $\sum_{i \in 7} Ai$ is a sum of $Ai$ over all biomarkers $i$ in the first group, $\sum_{j \in 1} Aj$ is a sum of $Aj$ over all biomarkers $j$ in the second group, and b is a constant, preferably selected from the range from 1, 3, 5, 8, or 10 to 20, 50, 100, 500, or 10000, alternatively selected from the range from 1 to 9, 15 to 200, 30 to 600, 800 to 1500, or combinations thereof.

## Computer-aided System and computer readable medium of Identifying a Biomarker

According to the present invention, a computer-aided system helpful in practicing the method of the present invention is provided. The present computer-aided system for assessing whether a subject mammal has a target condition comprises:

a) a sampling section configured for sampling an oral cavity sample of the subject mammal, wherein the sampled oral cavity sample is capable of containing:

i) a first group of biomarkers each having a higher abundance in oral cavities of a set of test mammals with said target condition compared to oral cavities of a set of test mammals without said target condition; and
ii) a second group of biomarkers each having a lower abundance in the oral cavities of the set of test mammals with said target condition compared to the oral cavities of the set of test mammals without said target condition;

b) a measuring section in communication with the sampling section, wherein said measuring section is configured for measuring the sampled oral cavity sample to obtain abundances of the first group and the second group of biomarkers in the sampled oral cavity sample; and
c) a computing section in communication with the measuring section, wherein said computing section stores a function of abundances of the first group of biomarkers and abundances of the second group of biomarkers that is useful for assessing whether the subject mammal has the target condition, and wherein the computing section is configured for applying the function to the obtained abundances of the first group and the second group of biomarkers in the sampled oral cavity sample to assess whether the subject mammal has the target condition.

The sampling section may comprise one or more devices in the form selected from the group consisting of a spoon, a cotton swab, a blade, a brush, a probe, and any combination thereof. In a specific embodiment, the sampling section comprises a sterile cotton swab, and the sampling is accomplished by gently rubbing exposed tooth surfaces with the sterile cotton swab.

In a specific embodiment, the present system can comprise a sample storage section for storing samples. If the samples collected from the sampling section are not to be used immediately, it is recommended to store them in the sample storage section. In a further specific embodiment, the sample storage has a temperature adjustment unit which can provide the sample storage section with a wide range of storing temperature, preferably below 30°C and more preferably below 0°C. In a preferred embodiment, the sample storage section provides a storing temperature of below -20°C, preferably below -50°C, more preferably below -70°C, and most preferably below -90°C.

The measuring section may comprise a sub-section performing one or more methods selected from the group consisting of 16S rRNA analysis, genetic fingerprinting, clone library method, denaturing- or temperature-gradient gel electrophoresis, random amplified polymorphic DNA, DNA amplification fingerprinting, amplified ribosomal DNA restriction analysis, DNA microarrays, fluorescence *in situ* hybridization, DNA-DNA hybridization, metagenomics, metaproteomics, metatranscriptomics, proteogenomics, Luria-Bertani medium isolation technique, Nutrient Agar isolation technique, Tryptic Soy Agar isolation technique, and any combination thereof.

The computing section can be in any form. For example, it can be a personal computer or a portable device which comprises a computing program. According to a specific embodiment, the computing section comprises:

i) a memory module for storing the function;
ii) an input module in communication with the measuring section, wherein the input module is for inputting the obtained abundances of the first group and the second group of biomarkers in the sampled oral cavity sample;
iii) a data processing module in communication with the memory module and the input module, wherein the data processing module is configured for applying the function to the inputted abundances of the first group and the second group of biomarkers in the sampled oral cavity sample; and
iv) an output module in communication with the data processing module, wherein the output module is for outputting whether the subject mammal has the target condition.

In a specific embodiment, the sampling section, the measuring section, and the computing section, alone or in any combination, can be implemented as a computer program product comprising computer executable instructions embodied in a computer readable medium.

In a further specific embodiment, the present invention provides a computer-readable medium for assessing whether a subject mammal has a target condition, comprising:

a) a memory storing a function of abundances of a first group of biomarkers and abundances of a second group of biomarkers that is useful for assessing whether the subject mammal has the target condition, wherein

each of the first group of biomarkers has a higher abundance in oral cavities of a set of test mammals with said target condition compared to oral cavities of a set of test mammals without said target condition, and each of the second group of biomarkers has a lower abundance in the oral cavities of the set of test mammals with said target condition compared to the oral cavities of the set of test mammals without said target condition; and

b) a computer code comprising instructions for applying the function to a data set obtained from the subject mammal, wherein the data set comprises abundances of the first group and the second group of biomarkers in an oral cavity sample of the subject mammal, assessing whether the subject mammal has the target condition.

Exemplary computer readable media include chip memory devices, disk memory devices, flash memory devices, programmable logic devices, application specific integrated circuits, downloadable electrical signals, and the like. In addition, a computer program product suitable for the present invention may be located on a single device or computing platform or may be distributed across multiple devices or computing platforms.

As necessary, one or more of the sections as stated above can be compacted into a large-size apparatus or a small-size portable device.

EXAMPLES

The examples herein are meant to exemplify the present invention but are not used to limit or otherwise define the scope of the present invention.

**List of Acronyms:**

NG: naturally occurring gingivitis
EG: experimental gingivitis
MGI: Modified/Mazza Gingival Index
BOP: Bleeding on Probing
MiGs: Microbial indices of Gingivitis
RPM: retrogression-progression model
PAM: Partitioning Around Medoids (clustering algorithm)
PCA: Principal Component Analysis
PCoA: Principal Coordinates Analysis
FDR: False Discovery Rate
COG: Clustering of Orthologous Groups
MiG: microbial index of gingivitis
MiG-S: microbial index of gingivitis sensitivity
ROC: receiver operating characteristic
CI: confidence interval
KO: KEGG Ortholog

**Target Conditions and Biomarkers**

A retrogression-progression model (RPM) is designed to simulate the development of gingivitis in human population to find biomarkers for gingivitis-related target conditions. Fifty human adults undergo a controlled temporal transition from naturally-occurring gingivitis ("NG") at Day -21 to healthy gingivae at Day 0 ("Baseline", as control status), then back to a state of experimental gingivitis ("EG") at Day 21. For each host, the structure of the plaque microbiota is measured at the three time points along the RPM: NG, Baseline and EG, thus allowing insight into dynamics. Taxonomic structures of the plaque microbiota are determined by pyrosequencing of 16S rRNA genes.

Fig. 1A illustrates a design of longitudinal study simulating gingivitis development in human population. Experiments are conducted at Procter & Gamble (Beijing) Technology Co., Ltd. Oral Care Department, with approval from the P&G Beijing Technical Center (China) Institutional Review Board and in accordance with the World Medical Association Declaration of Helsinki (1996 amendment). ICH Guidelines for Good Clinical Practice (GCP) are followed. Fifty subjects are recruited from the Beijing area. Voluntary informed consent is obtained.

Individuals meeting the following criteria are included: be at least 18 years of age; possess a minimum of 12 natural anterior teeth; have at least 5 bleeding sites as measured by Mazza Gingival Index (MGI) at initial visit (Day -21); have gingivitis but not periodontitis; be in good general health as determined by the Investigator/designee based on a review of the medical history/update for participation in the study. Exclusion criteria for individuals includes: severe periodontal disease, as characterized by purulent exudates, generalized mobility, and/or severe recession; any condition which requires antibiotic premedication for the administration of a dental prophylaxis; self-reported pregnancy or intent to become pregnant during the course of the study and nursing females; atypical discoloration or pigmentation in the gingival tissue; fixed facial orthodontic appliances; atypical discoloration or pigmentation in the gingival tissue; use of antibiotics any time during the study; any diseases or conditions that could be expected to interfere with the subject safely completing the study. Clinical parameters for each subject are measured per week across the whole study. Individuals that fell into the exclusion criteria at any time point are excluded from study participation.

The RPM includes three phases.

Phase I, Oral Hygiene Phase (Day -21 to Day 0): Gingivitis examinations using Mazza gingival index are conducted at -21, -14, -7 and 0 days. After receiving a dental prophylaxis (super and sub gingival prophylaxis) and tooth polishing, each subject is instructed to return to the site twice daily at which time they brush under supervision using Mei Li Liang Jie manual toothbrush (Crest, Made in China) for three minutes with a currently marketed anti-cavity dentifrice without any marked anti-microbial actives and then use the floss to clean the dental interproximal area. This brushing regimen is followed for the next 21 days while recording MGI for each subject each visit. During the Oral Hygiene Phase, subjects receive up to three dental prophylaxes if the subjects bleeding sites are more than 1.

Phase II, Experimental Gingivitis Phase (Day 0 to Day 21): During this phase, subjects do not have any oral hygiene practice including brushing, mouth rinsing with any products, flossing and dental prophylaxis. Subjects also receive a gingivitis exam at days 7, 14 and 21 of the Experimental Gingivitis Phase.

Phase III, Recovery Phase: Subjects are instructed to return to the site twice daily at which time they brush under supervision using products and techniques in Phase I. Subjects receive a dental prophylaxis during the Recovery Phase and the subjects also received gingivitis exam, inclusive of measured bleeding sites, to document and confirm that they have been returned to equivalent or preferably better health than when they enter the study. If needed, subjects receivean additional prophylaxis and are monitored until deemed healthy.

BOP frequency and mean MGI, as clinical parameters, are recorded for each subject. MGI measures both the signs of inflammation and the degree of the severity of bleeding. Specifically, probing is performed by a dentist on the mesiobuccal and the distolingual of each tooth, for a maximum of 56 sites. Scores range from 0 to 5, with 0 assigned for normal appearing and healthy gingival up to a score of 5 for spontaneous bleeding (without provocation). MGI of all subjects are measured by the same well-trained dentist to reduce technical variation.

Fig. 1B shows values of the above clinical parameters for 50 subjects cross the study. In Fig. 1B, boxes represent the interquartile range (IQR) and the lines inside represent the median. Whiskers denote the lowest and highest values within 1.5x IQR. At -21 day, all subjects exhibit a certain level of gingival inflammation that represents the state of naturally occurring gingivitis ("NG") with BOP ranging from 5 to 27 and average MGI from 1.18 to 2.24. These subjects then undergo rigorous oral hygiene practice for three weeks, which results in a greatly reduced BOP and MGI (Median BOP and MGI are 1.00 and 1.02 respectively) at 0 day ("Baseline") that represents a healthy gum state. Then the hosts further undergo an oral hygiene program for gingivitis induction for three weeks that results in significantly increased BOP (median 23) and MGI (median 2.11) representing the state of experimental gingivitis ("EG").

*Supragingival plaque sampling* Supragingival plaque samples from each subject are collected at Day -21, Day 0 and Day 21 following the procedures below. Subjects do not have oral hygiene practice including tooth brushing, flossing, mouth rinsing before sampling. Samples are collected after 2 hours food or drink (except water) intake. After MGI examination, each subject rinses their mouth with 50ml sterilized water. After MGI examination 15 minutes, plaque along the gumline within 2 mm depth are collected with Gracey curette by qualified dentists. For each subject, plaque samples are collected for all teeth in two different quadrants (1 and 3 or 2 and 4) and pooled together in one tube. Plaques on the Gracey curette are collected via swabbing with a sterilized cotton swab. The tips of swab are put into 0.6 ml TE20 buffer (20 mM Tris-HCl PH 8.0, 2 mM EDTA (ethylenediaminetetraacetic acid)). Before isolating DNA, all samples are stored under -70°C.

**Plaque DNA extraction protocol** Total DNA is extracted from Human Dental plaque following Dr. Larry Fernery's protocol with minor modifications (Ravel J, et al. (2011) Vaginal microbiome of reproductive-age women. Proc. Natl. Acad. Sci. U. S. A. 108:4680-4687). In general, frozen samples are thawed on ice before DNA isolation experiment. The original sample (250 μl) is transferred into a clean Bead-Beating-Tube (2ml Eppendorf tube). Sample suspensions are kept on ice while a Lytic-Enzyme Cocktail is prepared. Freshly prepared Lytic-Enzyme-Cocktail Master-Mix (100ul; containing 50 μl Lysozyme∼500KU=10mg/ml, 6 μl Mutanolysin, 25 KU/ml, 3 μl Lysostaphin, 4000 U/ml in 20 mM sodium acetate and 41 μl TE buffer) is added to all samples and incubated at 37°C for 45 min. To the lysate mix 750 mg cleaned and dry 0.1mm diameter Zirconia-Silica-Beads is added. Samples are subjected to bead beating for 2 minutes at room temperature in a Qiagen TissueLyser LT (36 oscillations/ second). One hundred and eighty μl of the crude lysate are transferred into a new tube and DNA isolated by Qiacube using DNeasy® Blood & Tissue Mini Kits.

**Bacterial 16S rRNA gene amplicon sequencing** 150 plaque samples are obtained and analyzed from 50 individuals each of whom provides samples at the three timepoints of NG (Day -21), Baseline (Day 0) and EG (Day +21). Barcoded 16S rDNA amplicon sequencing using 454 Titanium yields a total of 3,181,659 raw reads, resulting in totally 1,093,922 processed reads (i.e., reads after quality assessment and control measures). The number of processed reads per sample ranges from 437 to 28, 456, with an average 7293 reads per sample. All sequences are deposited at Sequence Read Archive under Accession ID SRA058763.

**Comparing the phylogenetic structures of plaque microbiota** PCA analysis is first performed in R using the ade4 package (Dray S & Dufour AB (2007) The ade4 package: Implementing the duality diagram for ecologists. Journal of Statistical Software 22(4):1-20) to visualize the difference of microbial community structure among different time points. Procrustes analysis attempts to stretch and rotate the points in one matrix, such as points obtained by PCA, to be as close as possible to points in the other matrix, thus preserving the relative distances between points within each matrix. Simple Procruste rotation in R using the ade4 package between two subsets of transformed data (i.e. data matrix of first-four principal components of NG-baseline, EG-baseline and NG-EG) is performed to test the degree of difference among different time points for the microbiota of the cohort.

Principal coordinates analysis (PCoA) is also performed to confirm the difference of microbiota structure between populations of gingivitis and health. In each sample, representative sequences from each OTU (operational taxonomic unit) are chosen by selecting the longest sequence based on UCLUST (Edgar RC (2010). Search and clustering orders of magnitude faster than BLAST. Bioinformatics 26(19):2460-2461). Each sequence is assigned to its closest relative in the phylogeny in CORE (Griffen AL, et al. (2011) CORE: a phylogenetically-curated 16S rDNA database of the core oral microbiome. PLoS One 6(4):e19051) using BLAST's megablast. The resulted sample ID (identification) mapping file and category mapping file are used as inputs to FastUniFrac (Hamady M, Lozupone C, & Knight R (2010) Fast UniFrac: facilitating high-throughput phylogenetic analyses of microbial communities including analysis of pyrosequencing and PhyloChip data. ISME J 4(1):17-27), which allows pairwise comparisons of inter-community distances based on the fraction of evolutionary history that separates the organisms. These distances are then clustered to reduce dimensionality using PCoA, where the principal coordinates (PC) describe in descending order the degree of variation that each of the axes in the new space explains. In addition, ThetaYC-based community structure comparisons are performed using MOTHUR (Schloss PD, Gevers D, & Westcott SL (2011) Reducing the effects of PCR (Polymerase Chain Reaction) amplification and sequencing artifacts on 16S rRNA-based studies. PLoS One 6(12):e27310). ThetaYC measures the structural dissimilarity between two communities. A matrix of pairwise thetaYC-based distances among all samples is created for clustering and PCoA analysis.

**Statistical analysis** To test the structural heterogeneity of microbiota, clustering among the plaque microbiota is performed by partitioning around medoids (PAM) using Jensen-Shannon divergence (JSD) of the normalized genus (or OTU) abundance. The optimal number of clusters is chosen based on the maximum of the silhouette index.

PCA analysis is then performed in R using the ade4 package to visualize the clustering based on PAM. Prior to the analysis, the data are sample-size normalized and very low abundant genera are removed (if their average abundance across all samples is below 0.1%) to decrease noise. Bacterial genera that exhibited the highest correlation to PC1 are identified and highlighted.

**Results** For each of the 150 plaque microbiota, bacterial phyla, genera and species are identified and their relative abundance quantified via taxonomic assignment against reference databases (CORE (Griffen AL, et al. (2011) CORE: a phylogenetically-curated 16S rDNA database of the core oral microbiome. PLoS One 6(4):e19051)).

At the phylum level, nearly all sequences are from 13 bacterial phyla, including six predominant bacterial phyla commonly encountered in the oral cavity: *Firmicutes, Proteobacteria, Bacteroidetes, Actinobacteria, Fusobacteria* and *TM7* (each with average relative abundance > 1% at least one timepoint). Between the gingivitis states (NG and EG) and the healthy gingival state (Baseline), significant difference ($p < 0.01$; paired *t*-test) are found in five predominate phyla: *Actinobacteria, Firmicutes, TM7, Bacteroidetes* and *Fusobacteria. A* temporal shift of community-structure along the NG-Baseline-EG progression is apparent, characterized by the elevated relative abundance of *Actinobacteria* and *Firmicutes* at Baseline, and that of *TM7, Bacteroidetes* and *Fusobacteria* at NG and EG.

At the genus level, 27 bacterial genera (each with average relative abundance >0.1% at least one time point) are differentially distributed ($p<0.05$, paired $t$-test; FDR (false discovery rate) $q<0.2$) between Baseline and gingivitis (both NG and EG). Among them, five (*Streptococcus, Rothia, Actinomyces, Haemophilus* and *Lautropia*) show elevated abundance at Baseline, while 22 (*Leptotrichia, Prevotella, Fusobacterium,* TM7, *Porphyromonas*, *Tannerella, Selenomonas, Uncultured_Lachnospiraceae*, unclassified_Comamonadaceae, *Peptococcus, Aggregatibacter, Catonella, Treponema,* SRI, *Campylobacter, Eubacterium, Peptostreptococcus, unclassified_Bacteroidaceae, Solobacterium, Johnsonella, Oribacterium,* and *unclassified_Veillonellaceae*) are enriched in both NG and EG. Fig. 2 shows these 27 genus-level bacterial biomarkers that are believed to denote gum health and Gingivitis (for both naturally occurring gingivitis and experimental gingivitis). Relative abundance of identified oral bacteria in microbial community at different stages is also displayed. These bacterial taxa can potentially serve as disease markers.

Along the RPM, different bacterial species within the same genus usually exhibited identical patterns of relative-abundance change, except for several species of *Capnocytophaga*, *Actinomyces* and *Streptococcus.*

The projected coordinate of a given microbiota on the PC1 appears to capture the gradient-like heterogeneity and development of microbiota structure along disease retrogression and progression, as changes in PC1 within subjects and across cohorts are largely consistent with the structural segregation between healthy and diseased microbiota (see Figs. 3A and 3B). Moreover, the relative order of microbiota along PC1 defined using all 150 samples is similar to those defined using healthy-only, NG-only or EG-only microbiota alone (Spearman correlation; All vs Healthy-only: $rho$=0.95, $p<0.001$; All vs NG: $rho$= 0.97, $p<0.001$; All vs EG: $rho$= 0.97, $p<0.001$). Therefore PC1 appears to be the primary descriptor and thus a good proxy for quantitatively measuring the development of the microbiota in both RPM-segments (e.g. NG-to-Baseline and Baseline-to-EG).

For the 50 hosts along RPM, 15 bacterial genera are found to be the drivers of microbiota heterogeneity along PC1, as their gradients in abundance are significantly correlated with the coordinates of their corresponding samples on PC1 (Spearman $rho$>0.7, FDR $q<0.2$), as shown in Table 1 below. These drivers include *Rothia, Haemophilus, Prevotella, Leptotrichia, Fusobacterium, Selenomonas,* uncultured *Lachnospiraceae,* TM7, *Tannerella, Peptococcus, Peptostreptococcus, Catonella, Treponema, Solobacterium* and unclassified Bacteroidaceae.

Table 1. Oral bacterial that shows significant correlation with PC1

| Genus | Rho value |
|---|---|
| *Rothia* | -0.76 |
| *Haemophilus* | -0.7 |
| *Prevotella* | 0.85 |
| *Leptotrichia* | 0.81 |
| *Fusobacterium* | 0.71 |
| *Selenomonas* | 0.85 |
| uncultured *Lachnospiraceae* | 0.83 |
| TM7 | 0.81 |
| *Tannerella* | 0.74 |
| *Peptococcus* | 0.82 |
| *Peptostreptococcus* | 0.73 |
| *Catonella* | 0.73 |
| *Treponema* | 0.82 |
| *Solobacterium* | 0.72 |
| unclassified Bacteroidaceae | 0.72 |

Two of the 15 genera, *Rothia* and *Haemophilus,* decrease in relative abundance along PC1 ("negative drivers"), while the other 13 increase along PC1 ("positive drivers").

Among the 50 subjects, most hosts exhibit a largely consistent microbiota structure during the disease progression from NG to EG (see Fig. 4A). Although NG-Baseline (or Baseline-EG) PCI-changes vary considerably among the 50-host cohort, the rate of microbiota change NG-Baseline and that of microbiota change Baseline-EG are largely similar within each subject. The persistence of disease outcome as well as microbiota structure for majority of the hosts in EG (as compared to NG) suggest the presence of host-dependent (and likely personal) factors in determining the susceptibility to gingivitis reoccurrence in natural human populations.

PCA based on within-subject changes of both microbiota (ΔPC1 at NG-to-Baseline and ΔPC1 at Baseline-to-EG) and clinical symptom (ΔMGI at NG-to-Baseline and ΔMGI at Baseline-to-EG) along RPM reveal the divergence of disease susceptibility among the 50 hosts.

As shown in Fig. 4B, all hosts in the 50-member cohort are plotted on the first two principle components of the PCA based on the change profiles of microbiota and MGI. The histogram and the kernel density plot (solid line) describing distribution of the 50 hosts along the principle component of the PCA are shown. The vertical dash line divides the 50 hosts into Type-I (dots) and Type-II (triangles). The four variables as main contributors to these clusters are determined and plotted by their loadings in these two principle components. "a" denotes ΔPC1 (NG-Baseline); "b" denotes ΔMGI (NG-Baseline); "c" denotes ΔPC1(Baseline-EG); and "d" denotes ΔMGI(Baseline-EG).

The distribution pattern of the 50 hosts suggests a bimodal distribution ($p = 0.74$ for the hypothesis of non-bimodal distribution based on Hartigans' dip test for unimodality), where a discriminating line can be drawn to divide the hosts into two types which we designated as less gingivitis-sensitive Type-I (17 individuals) and gingivitis-sensitive Type-II (33 individuals). Type-II hosts are characterized by more acute changes in both microbiota structure than Type-I hosts (see Fig. 4A and Fig. 4C). For an average Type-II host, the PC1-change rate along RPM is 0.33 per day, which are 2.21 fold of an average Type-I host (see Fig. 4C).

At both NG and EG, there are significant relationship between gingivitis-sensitive types and the relative abundance of certain taxa ($p<0.05$, Wilcoxon rank-sum test), which include *Abiotrophia, Selenomonas, uncultured Lachnospiraceae, Peptococcus,* unclassified *Bacteroidaceae, Peptostreptococcus, Oribacterium* and *Veillonellaceae*; these taxa are all enriched in Type-II hosts as compared to Type I hosts, except *Abiotrophia* which is enriched in Type-I (see Fig. 4D). Most (five) of these Type-II-hosts associated genera are among the 15 PC1-drivers.

## Function Formulation and Assessment

The 50-host cohort is used as a training set for function formulation, while an additional 41 human subjects with naturally occurring gingivitis are recruited and then each sampled at both NG and Baseline (thus 82 additional microbiota samples are sequenced) for assessment.

(1) **MiG27:** The inventors formulate a function as a "microbial index of gingivitis" (MiG) based on the relative abundance of the 27 bacterial markers that are distinguished between the Baseline stage and the gingivitis stages (NG and EG) in the 50-host cohort (MiG27), via the following equation:

$$MiG27 = (\frac{\sum_{i=22} abundance(g_{Gingivitis-enriched})_i}{22} - \frac{\sum_{j=5} abundance(g_{Health-enriched})_j}{5}) \times 10$$

In the 50-host cohort, this index is highly correlated with MGI during both NG-to-Baseline ($p<0.001$, Student's $t$-test) and Baseline-to-EG ($p<0.001$, Student's $t$-test): the area under the receiver operating characteristic (ROC) curve is 99.52% (95% confidence interval: 98.77%-99.52%) at NG-to-Baseline and 99.84% (95% confidence interval: 99.53%-99.84%) at baseline-to-EG.

With MiG27, the inventors predict gingivitis status of the 41 hosts in the new cohort using their NG microbiota. Fig. 5 shows the MiG27 indices of the additional cohort of 41 hosts. Boxes represent the IQR and the lines inside represent the median. Whiskers denote the lowest and highest values within 1.5x IQR. The MiG27 between NG (MGI>1.18) and Baseline (MGI<1.12) is significantly different ($p<0.001$, paired $t$-test, $t$ statistic= 22.3), e.g. the top 27 samples with the highest MiG27 are all correctly classified as gingivitis. The overall accuracy of prediction (based on Linear Discriminant Analysis) for diseased state versus healthy state is 94% (i.e., an error rate of 6.1%) (see Table 2 below).

(2) **MiG15:** To assess diseased severity of gingivitis, MiG15, which is based on the relative abundance of 15 bacterial genera that drive the structural heterogeneity of microbiota along PC1, is derived. The MiG15 of a given microbiota is calculated via the following equation:

$$MiG15 = (\frac{\sum_{i=13} abundance(g_{High\_PC1-enriched})_i}{13} - \frac{\sum_{j=2} abundance(g_{Low\_PC1-enriched})_j}{2}) \times 10$$

The inventors then regress the relative PC1-values (**Y**: the development of gingivitis) on MiG15 (**X**) using linear regression. The formula for prediction is: **$Y=-0.97-4.62X$.** This revised function is able to account for 60% of variance in PC1 location in the 50-host cohort. This function on disease severity is used to assess the NG microbiota in the 41-host cohort. Fig. 6 shows the MiG15 indices of the additional cohort of 41 hosts. Boxes represent the interquartile range (IQR) and the lines inside represent the median. Whiskers denote the lowest and highest values within 1.5x IQR. The heatmap indicates the ability of MiG15 to discriminate healthy and gingivitis status of hosts. MiG15 shows significant correlation with mean MGI for each subjects at NG ($p$ <0.05, spearman correlation). Categorization of both predicted values and test values (of PC1) into three quantiles reveals an error rate of prediction at 24.4% (see Table 2 below). Therefore, the MiG15-based function is able to predict the gingivitis severity in human hosts as defined by PC1 at approximately 75% accuracy.

(3) **MiG-Sensitivity (MiG-S):** The inventors further derive a "microbial index of gingivitis sensitivity" (MiG-S) based on the relative abundance of the eight bacterial markers that distinguish between the Type-I and Type-II in the 50-host cohort at NG (MiG-S), via the following equation:

$$MiG-S = (\frac{\sum_{i=7} abundance(g_{TypeII-enriched})_i}{7} - \frac{\sum_{j=1} abundance(g_{TypeI-enriched})_j}{1}) \times 10$$

In the 50-host cohort, this index is highly correlated with types ($p$<0.05, Wilcoxon rank-sum test): the area under the ROC curve is 74.0% (95% confidence interval: 60.2%-74.0%) (see Fig. 7), suggesting an up to 74.0% accuracy of predicting gingivitis-sensitivity host-types. Table 2 below shows the same result.

Table 2. Predictive Functions of Human Gingivitis based on Plaque Microbiota

|  | Error rate | MiG27 | MiG15 | MiG-S |
|---|---|---|---|---|
| Clinical status | Health vs. Gingivitis | 6.1% | 6.1% | - |
| Categorized status of gingivitis | Based on MGI | 41.5% | 41.5% | - |
|  | Based on PC1 | 24.4% | 24.4% | - |
| Gingivitis sensitivity of the host | Based on change-pattern of PC1 and MGI | - | - | 26.0% |

**Discussion**

The identified microbial drivers of gingivitis development and susceptibility and the formulated functions based on the same provide novel opportunities to improve clinical practice. In gingivitis, the gingival tissue exhibits color change, contour alteration, increased sulcular exudates and bleeding upon provocation. Based on one or more of such host symptoms, current gingival indices proposed or practiced are subjective, prone to human bias and error and difficult to reproduce, as such indices are heavily dependent upon the human examiner's visual observation and individual judgment. For example, despite its prevalent use in clinical practice, MGI in being a subjective measure of gingivitis severity can be of poor reproductivity among different examiners. Moreover, as symptom of gingivitis can vary greatly among different teeth (and even probing points), testing two probing sites for each of the 28 teeth for each patient can be time- and labor-intensive. These drawbacks have collectively confounded cross-examiner and cross-patients analysis of gingivitis. According to the present invention, the inventors develop and validate an alternative and likely complementary measure for gingivitis that is based on quantitative analysis of plaque microbiota. The proposed MiG-based predictive functions are able to predict diseased microbiota at 95% accuracy, distinguish different disease-stages with 75% accuracy, and potentially predict disease sensitivity. MiGs can thus serve as more sensitive, reliable and objective measures of gum health and gingivitis susceptibility and thus contribute to the diagnosis, prognosis and intervention of gum diseases.

Unless otherwise indicated, all percentages, ratios, and proportions are calculated based on weight of the total composition. All temperatures are in degrees Celsius (°C) unless otherwise indicated. All measurements made are at 25°C, unless otherwise designated. All component or composition levels are in reference to the active level of that component or composition, and are exclusive of impurities, for example, residual solvents or by-products, which may be present in commercially available sources.

It should be understood that every maximum numerical limitation given throughout this specification includes every lower numerical limitation, as if such lower numerical limitations are expressly written herein. Every minimum numerical limitation given throughout this specification will include every higher numerical limitation, as if such higher numerical limitations are expressly written herein. Every numerical range given throughout this specification will include every narrower numerical range that falls within such broader numerical range, as if such narrower numerical ranges are all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Every document cited herein, including any cross referenced or related patent or application is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method of assessing whether a subject mammal has a target condition, comprising the steps:

   a) defining the target condition, wherein the target condition is selected from the group consisting of a disease, severity of a disease, sensitivity to a disease, and combinations thereof and the disease is a microbe-related disease;

   defining a first group of biomarkers each having a higher abundance in oral cavities of a set of test mammals with said target condition compared to oral cavities of a set of test mammals without said target condition, wherein the first group of biomarkers are bacterial genera selected from the group consisting of *Leptotrichia, Prevotella, Fusobacterium,* TM7, *Porphyromonas*, *Tannerella, Selenomonas, Lachnospiraceae, Comamonadaceae, Peptococcus*, *Aggregatibacter, Catonella, Treponema,* SRI, *Campylobacter, Eubacterium, Peptostreptococcus, Bacteroidaceae, Solobacterium, Johnsonella, Oribacterium, Veillonellaceae,* and combinations thereof;

   b) defining a second group of biomarkers each having a lower abundance in the oral cavities of the set of test mammals with said target condition compared to the oral cavities of the set of test mammals without said target condition, wherein the second group of biomarkers are bacterial genera selected from the group consisting of *Streptococcus, Rothia, Actinomyces, Haemophilus, Lautropia,* and combinations thereof.;

   c) formulating a linear function of the abundances of the first group of biomarkers and the abundances of the second group of biomarkers that is useful for assessing whether the subject mammal has the target condition comprising a formula:

   $$f(Ai, Aj) = b\left(\frac{\sum_{i \in N} Ai}{N} - \frac{\sum_{j \in M} Aj}{M}\right)$$

   where $N$ is a total number of the biomarkers in the first group, $M$ is a total number of the biomarkers in the second group, $Ai$ is an abundance of each biomarker $i$ in the first group, $Aj$ is an abundance of each *biomarker* $j$ in the second group, $\sum_{i \in N} Ai$ is a sum of $Ai$ over all biomarkers $i$ in the first group, $\sum_{j \in M} Aj$ is a sum of $Aj$ over all biomarkers $j$ in the second group, and b is a constant;

   d) obtaining a sample from an oral cavity of the subject mammal, wherein the obtained sample is capable of containing the first group of biomarkers and the second group of biomarkers;

   e) measuring abundances of the first group of biomarkers in the obtained sample from the subject mammal;

   f) measuring abundances of the second group of biomarkers in the obtained sample from the subject mammal; and

   g) inputting the measured abundances of the first group and the second group of biomarkers into the formulated function to assess whether the subject mammal has the target condition.

2. The method according to claim 1, wherein the disease is selected from the group consisting of gingivitis, periodontitis, dental caries, halitosis, oral ulcer, premature birth, diabetes, respiratory disease, stroke, bacteremia and combinations thereof, and is preferably gingivitis.

3. The method according to claim 1, wherein the biomarkers are each independently selected from the group consisting of taxonomic categories of a bacterium, functional categories of a microbe, and combinations thereof, preferably selected from the group consisting of a bacterial phylum, a bacterial class, a bacterial family, a bacterial order, a bacterial genus, a bacterial species, a functional gene of a microbe, a gene ortholog group of a microbe, a motif of peptide or protein of a microbe, a conserved peptide or protein domain of a microbe, a none-coding nucleotide sequence of a microbe, and combinations thereof.

4. The method according to claim 1, wherein the target condition is gingivitis.

5. The method according to claim 4, wherein the function of the abundances of the first group of biomarkers and the abundances of the second group of biomarkers is:

$$f(Ai, Aj) = b(\frac{\sum_{i \in 22} Ai}{22} - \frac{\sum_{j \in 5} Aj}{5})$$

where $Ai$ is an abundance of each biomarker $i$ in the first group, $Aj$ is an abundance of each biomarker $j$ in the second group, $\sum_{i \in 22} Ai$ is a sum of $Ai$ over all biomarkers $i$ in the first group, $\sum_{j \in 5} Aj$ is a sum of $Aj$ over all biomarkers $j$ in the second group, and b is a constant, preferably 10.

6. The method according to claim 1, wherein the first group of biomarkers are bacterial genera selected from the group consisting of *Prevotella, Leptotrichia, Fusobacterium, Selenomonas, Lachnospiraceae, TM7, Tannerella, Peptococcus, Peptostreptococcus, Catonella, Treponema, Solobacterium, Bacteroidaceae,* and combinations thereof; and the second group of biomarkers are bacterial genera selected from the group consisting of *Rothia, Haemophilus,* and combination thereof.

7. The method according to claim 6, wherein the target condition is severity of gingivitis.

8. The method according to claim 7, wherein the function of the abundances of the first group of biomarkers and the abundances of the second group of biomarkers is:

$$f(Ai, Aj) = b(\frac{\sum_{i \in 13} Ai}{13} - \frac{\sum_{j \in 2} Aj}{2})$$

where $Ai$ is an abundance of each biomarker $i$ in the first group, $Aj$ is an abundance of each biomarker $j$ in the second group, $\sum_{i \in 13} Ai$ is a sum of $Ai$ over all biomarkers $i$ in the first group, $\sum_{j \in 2} Aj$ is a sum of $Aj$ over all biomarkers $j$ in the second group, and b is a constant, preferably 10.

9. The method according to claim 1, wherein the first group of biomarkers are bacterial genera selected from the group consisting of *Selenomonas, Lachnospiraceae, Peptococcus, Bacteroidaceae, Peptostreptococcus, Oribacterium, Veillonellaceae* and combinations thereof; and the second group of biomarkers is a bacterial genus of *Abiotrophia.*

10. The method according to claim 9, wherein the target condition is sensitivity to gingivitis.

11. The method according to claim 10, wherein the function of the abundances of the first group of biomarkers and the abundances of the second group of biomarkers is:

$$f(Ai, Aj) = b(\frac{\sum_{i \in 7} Ai}{7} - \frac{\sum_{j \in 1} Aj}{1})$$

where $Ai$ is an abundance of each biomarker $i$ in the first group, $Aj$ is an abundance of each *biomarker j* in the second group, $\sum_{i \in 7} Ai$ is a sum of $Ai$ over all biomarkers $i$ in the first group, $\sum_{j \in 1} Aj$ is a sum of $Aj$ over all biomarkers $j$ in the second group, and b is a constant, preferably 10.

12. The method according to claim 1, wherein the sample is selected from the group consisting of a salivary sample, a supragingival plaque sample, a subgingival plaque sample, a tooth plaque sample, and combinations thereof.

**13.** The method according to claim 1, wherein the abundances of the first and second groups of biomarkers are measured by a method selecting from the group consisting of 16S rRNA analysis, metagenomics, and combination thereof.

**14.** A computer-aided system for performing the method according to anyone of claims 1 to 13.

**Patentansprüche**

**1.** Verfahren zur Feststellung, ob ein untersuchtes Säugetier eine festzustellende Verfassung aufweist, die folgenden Schritte umfassend:

a) Definieren der festzustellenden Verfassung, wobei die festzustellende Verfassung ausgewählt ist aus der Gruppe bestehend aus einer Krankheit, der Schwere einer Krankheit, der Anfälligkeit für eine Krankheit und Kombinationen davon, und die Krankheit eine mit Mikroben in Beziehung stehende Krankheit ist; Definieren einer ersten Gruppe von Biomarkern, die in Mundhöhlen einer Gruppe von getesteten Säugetieren mit der festzustellenden Verfassung im Vergleich zu Mundhöhlen einer Gruppe von getesteten Säugetieren ohne die festzustellende Verfassung jeweils in größerer Menge vorkommen, wobei die erste Gruppe von Biomarkern Bakteriengattungen sind, die ausgewählt sind aus der Gruppe bestehend aus *Leptotrichia, Prevotella, Fusobacterium,* TM7, *Porphyromonas*, *Tannerella*, *Selenomonas*, *Lachnospiraceae, Comamonadaceae, Peptococcus, Aggregatibacter, Catonella, Treponema,* SR1, *Campylobacter, Eubacterium, Peptostreptococcus, Bacteroidaceae, Solobacterium, Johnsonella, Oribacterium, Veillonellaceae* und Kombinationen davon;
b) Definieren einer zweiten Gruppe von Biomarkern, die in den Mundhöhlen der Gruppe getesteter Säugetiere mit der festzustellenden Verfassung im Vergleich zu Mundhöhlen der Gruppe getesteter Säugetiere ohne die festzustellende Verfassung jeweils in geringerer Menge vorkommen, wobei die zweite Gruppe von Biomarkern Bakteriengattungen sind, die ausgewählt sind aus der Gruppe bestehend aus *Streptococcus, Rothia, Actinomyces, Haemophilus, Lautropia* und Kombinationen davon;
c) Formulieren einer linearen Funktion der Mengen der ersten Gruppe von Biomarkern und der Mengen der zweiten Gruppe von Biomarkern, die geeignet ist, um festzustellen, ob das untersuchte Säugetier die festzustellende Verfassung aufweist, umfassend eine Formel:

$$f(Ai, Aj) = b\left(\frac{\sum_{i \in N} Ai}{N} - \frac{\sum_{j \in M} Aj}{M}\right)$$

worin *N* eine Gesamtzahl der Biomarker in der ersten Gruppe ist, *M* eine Gesamtzahl der Biomarker in der zweiten Gruppe ist, *Ai* eine Menge jedes Biomarkers *i* in der ersten Gruppe ist, *Aj* eine Menge jedes Biomarkers *j* in der zweiten Gruppe ist, $\sum_{i \in N} Ai$ eine Summe von *Ai* über allen Biomarkern *i* in der ersten Gruppe ist, $\sum_{i \in M} Aj$ eine Summe von *Aj* über allen Biomarkern *j* in der zweiten Gruppe ist, und b eine Konstante ist;
d) Entnehmen einer Probe aus einer Mundhöhle des untersuchten Säugetiers, wobei in der entnommenen Probe die erste Gruppe von Biomarkern und die zweite Gruppe von Biomarkern enthalten sein können;
e) Messen der Mengen der ersten Gruppe von Biomarkern in der dem untersuchten Säugetier entnommenen Probe;
f) Messen der Mengen der zweiten Gruppe von Biomarkern in der dem untersuchten Säugetier entnommenen Probe; und
g) Einsetzen der gemessenen Mengen der ersten Gruppe und der zweiten Gruppe von Biomarkern in die formulierte Funktion, um festzustellen, ob das untersuchte Säugetier die festzustellende Verfassung aufweist.

**2.** Verfahren nach Anspruch 1, wobei die Krankheit ausgewählt ist aus der Gruppe bestehend aus Gingivitis, Parodontitis, Zahnkaries, Halitosis, Mundgeschwüren, Frühgeburtlichkeit, Diabetes, Atemwegserkrankung, Schlaganfall, Bakteriämie und Kombinationen davon, und vorzugsweise Gingivitis ist.

**3.** Verfahren nach Anspruch 1, wobei die Biomarker jeweils unabhängig ausgewählt sind aus der Gruppe bestehend aus taxonomischen Kategorien eines Bakteriums, funktionalen Kategorien einer Mikrobe und Kombinationen davon, und vorzugsweise ausgewählt sind aus der Gruppe bestehend aus einem Bakterienstamm, einer Bakterienklasse, einer Bakterienfamilie, einer Bakterienordnung, einer Bakteriengattung, einer Bakterienspezies, einem funktionellen Gen einer Mikrobe, einer genorthologen Gruppe einer Mikrobe, einem Motiv eines Peptids oder Proteins einer Mikrobe, einer konservierten Peptid- oder Proteindomäne einer Mikrobe, einer nicht-kodierenden Nukleotidsequenz einer Mikrobe und Kombinationen davon.

4.  Verfahren nach Anspruch 1, wobei die festzustellende Verfassung Gingivitis ist.

5.  Verfahren nach Anspruch 4, wobei die Funktion der Mengen der ersten Gruppe von Biomarkern und der Mengen der zweiten Gruppe von Biomarkern wie folgt ist:

$$f(Ai, Aj) = \mathrm{b}\left(\frac{\sum_{i \in 22} Ai}{22} - \frac{\sum_{j \in 5} Aj}{5}\right)$$

wobei *Ai* eine Menge jedes Biomarkers *i* in der ersten Gruppe ist, *Aj* eine Menge jedes Biomarkers *j* in der zweiten Gruppe ist, $\sum_{i \in 22} Ai$ eine Summe von *Ai* über allen Biomarkern *i* in der ersten Gruppe ist, $\sum_{j \in 5} Aj$ eine Summe von *Aj* über allen Biomarkern *j* in der zweiten Gruppe ist, und b eine Konstante, vorzugsweise 10, ist.

6.  Verfahren nach Anspruch 1, wobei die erste Gruppe von Biomarkern aus Bakteriengattungen besteht, die ausgewählt sind aus der Gruppe bestehend aus *Prevotella, Leptotrichia, Fusobacterium, Selenomonas*, *Lachnospiraceae,* TM7, *Tannerella*, *Peptococcus, Peptostreptococcus, Catonella, Treponema, Solobacterium, Bacteroidaceae* und Kombinationen davon; und die zweite Gruppe von Biomarkern aus Bakteriengattungen besteht, die ausgewählt sind aus der Gruppe bestehend aus *Rothia, Haemophilus* und Kombinationen davon.

7.  Verfahren nach Anspruch 6, wobei die festzustellende Verfassung die Schwere einer Gingivitis ist.

8.  Verfahren nach Anspruch 7, wobei die Funktion der Mengen der ersten Gruppe von Biomarkern und der Mengen der zweiten Gruppe von Biomarkern wie folgt ist:

$$f(Ai, Aj) = \mathrm{b}\left(\frac{\sum_{i \in 13} Ai}{13} - \frac{\sum_{j \in 2} Aj}{2}\right)$$

wobei *Ai* eine Menge jedes Biomarkers i in der ersten Gruppe ist, *Aj* eine Menge jedes Biomarkers *j* in der zweiten Gruppe ist, $\sum_{i \in 13} Ai$ eine Summe von *Ai* über allen Biomarkern *i* in der ersten Gruppe ist, $\sum_{j \in 2} Aj$ eine Summe von *Aj* über allen Biomarkern *j* in der zweiten Gruppe ist, und b eine Konstante, vorzugsweise 10, ist.

9.  Verfahren nach Anspruch 1, wobei die erste Gruppe von Biomarkern Bakteriengattungen sind, die ausgewählt sind aus der Gruppe bestehend aus *Selenomonas*, *Lachnospiraceae, Peptococcus, Bacteroidaceae, Peptostreptococcus, Oribacterium, Veillonellaceae* und Kombinationen davon; und die zweite Gruppe von Biomarkern eine Bakteriengattung *Abiotrophia* ist.

10. Verfahren nach Anspruch 9, wobei die festzustellende Verfassung die Anfälligkeit für Gingivitis ist.

11. Verfahren nach Anspruch 10, wobei die Funktion der Mengen der ersten Gruppe von Biomarkern und der Mengen der zweiten Gruppe von Biomarkern wie folgt ist:

$$f(Ai, Aj) = \mathrm{b}\left(\frac{\sum_{i \in 7} Ai}{7} - \frac{\sum_{j \in 1} Aj}{1}\right)$$

wobei *Ai* eine Menge jedes Biomarkers i in der ersten Gruppe ist, *Aj* eine Menge jedes Biomarkers *j* in der zweiten Gruppe ist, $\sum_{i \in 7} Ai$ eine Summe von *Ai* über allen Biomarkern *i* in der ersten Gruppe ist, $\sum_{j \in 1} Aj$ eine Summe von *Aj* über allen Biomarkern *j* in der zweiten Gruppe ist, und b eine Konstante, vorzugsweise 10, ist.

12. Verfahren nach Anspruch 1, wobei die Probe ausgewählt ist aus der Gruppe bestehend aus einer Speichelprobe, einer supragingivalen Zahnbelagsprobe, einer subgingivalen Zahnbelagsprobe, einer Zahnbelagsprobe und Kombinationen davon.

13. Verfahren nach Anspruch 1, wobei die Mengen der ersten und der zweiten Gruppe von Biomarkern anhand eines Verfahrens gemessen werden, das ausgewählt ist aus der Gruppe bestehend aus 16S rRNA-Analyse, Metagenomik und Kombinationen davon.

**14.** Computerunterstütztes System zur Durchführung des Verfahrens nach einem der Ansprüche 1 bis 13.


**Revendications**

**1.** Procédé permettant d'évaluer si un mammifère sujet présente une condition cible, comprenant les étapes consistant à :

a) définir la condition cible, dans lequel la condition cible est choisie dans le groupe constitué d'une maladie, une gravité d'une maladie, une sensibilité à une maladie, et leurs combinaisons et la maladie est une maladie liée à un microbe ;
définir un premier groupe de biomarqueurs ayant chacun une abondance plus élevée dans les cavités buccales d'un ensemble de mammifères de test présentant ladite condition cible par comparaison avec les cavités buccales d'un ensemble de mammifères de test dépourvus de ladite condition cible, dans lequel les biomarqueurs du premier groupe sont des genres bactériens choisis dans le groupe constitué de *Leptotrichia, Prevotella, Fusobacterium,* TM7, *Porphyromonas, Tannerella, Selenomonas, Lachnospiraceae, Comamonadaceae, Peptococcus, Aggregatibacter, Catonella, Treponema,* SR1, *Campylobacter, Eubacterium, Peptostreptococcus, Bacteroidaceae, Solobacterium, Johnsonella, Oribacterium, Veillonellaceae,* et leurs combinaisons ;
b) définir un deuxième groupe de biomarqueurs ayant chacun une abondance plus basse dans les cavités buccales de l'ensemble de mammifères de test présentant ladite condition cible par comparaison avec les cavités buccales de l'ensemble de mammifères de test dépourvus de ladite condition cible, dans lequel les biomarqueurs du deuxième groupe sont des genres bactériens choisis dans le groupe constitué de *Streptococcus, Rothia, Actinomyces, Haemophilus, Lautropia,* et leurs combinaisons ;
c) formuler une fonction linéaire des abondances du premier groupe de biomarqueurs et des abondances du deuxième groupe de biomarqueurs qui est utile pour évaluer si le mammifère sujet présente la condition cible, comprenant une formule :

$$f(Ai, Aj) = b\left(\frac{\sum_{i \in N} Ai}{N} - \frac{\sum_{j \in M} Aj}{M}\right)$$

où *N* est un nombre total des biomarqueurs dans le premier groupe, *M* est un nombre total des biomarqueurs dans le deuxième groupe, *Ai est* une abondance de chaque biomarqueur *i* dans le premier groupe, *Aj* est une abondance de chaque biomarqueur *j* dans le deuxième groupe, $\sum_{i \in N} Ai$ est une somme des *Ai* sur tous les biomarqueurs *i* dans le premier groupe, $\sum_{j \in M} Aj$ est une somme des *Aj* sur tous les biomarqueurs *j* dans le deuxième groupe, et b est une constante ;
d) obtenir un échantillon provenant d'une cavité buccale du mammifère sujet, dans lequel l'échantillon obtenu est susceptible de contenir le premier groupe de biomarqueurs et le deuxième groupe de biomarqueurs ;
e) mesurer les abondances du premier groupe de biomarqueurs dans l'échantillon obtenu auprès du mammifère sujet ;
f) mesurer les abondances du deuxième groupe de biomarqueurs dans l'échantillon obtenu auprès du mammifère sujet ; et
g) introduire les abondances mesurées du premier groupe et du deuxième groupe de biomarqueurs dans la fonction formulée pour évaluer si le mammifère sujet présente la condition cible.

**2.** Procédé selon la revendication 1, dans lequel la maladie est choisie dans le groupe constitué de gingivite, parodontite, carie dentaire, halitose, ulcère buccal, accouchement prématuré, diabète, maladie respiratoire, accident vasculaire cérébral, bactériémie et leurs combinaisons, et est de préférence la gingivite.

**3.** Procédé selon la revendication 1, dans lequel les biomarqueurs sont chacun indépendamment choisis parmi le groupe constitué de catégories taxonomiques d'une bactérie, catégories fonctionnelles d'un microbe, et leurs combinaisons, choisis de préférence dans le groupe constitué d'un phylum bactérien, une classe bactérienne, une famille bactérienne, un ordre bactérien, un genre bactérien, une espèce bactérienne, un gène fonctionnel d'un microbe, un groupe d'orthologues géniques d'un microbe, un motif de peptide ou protéine d'un microbe, un domaine peptidique ou protéinique conservé d'un microbe, une séquence nucléotidique non codante d'un microbe, et leurs combinaisons.

**4.** Procédé selon la revendication 1, dans lequel la condition cible est la gingivite.

**5.** Procédé selon la revendication 4, dans lequel la fonction des abondances du premier groupe de biomarqueurs et des abondances du deuxième groupe de biomarqueurs est :

$$f(Ai, Aj) = b\left(\frac{\sum_{i\in22} Ai}{22} - \frac{\sum_{j\in5} Aj}{5}\right)$$

où *Ai est* une abondance de chaque biomarqueur *i* dans le premier groupe, *Aj* est une abondance de chaque biomarqueur *j* dans le deuxième groupe, $\sum_{i\in22} Ai$ est une somme des *Ai* sur tous les biomarqueurs *i* dans le premier groupe, $\sum_{j\in5} Aj$ est une somme des *Aj* sur tous les biomarqueurs *j* dans le deuxième groupe, et b est une constante, de préférence 10.

**6.** Procédé selon la revendication 1, dans lequel les biomarqueurs du premier groupe sont des genres bactériens choisis dans le groupe constitué de *Prevotella, Leptotrichia, Fusobacterium, Selenomonas, Lachnospiraceae,* TM7, *Tannerella, Peptococcus, Peptostreptococcus*, *Catonella, Treponema, Solobacterium, Bacteroidaceae*, et leurs combinaisons ; et les biomarqueurs du deuxième groupe sont des genres bactériens choisis dans le groupe constitué de *Rothia, Haemophilus,* et une combinaison de ceux-ci.

**7.** Procédé selon la revendication 6, dans lequel la condition cible est une gravité de gingivite.

**8.** Procédé selon la revendication 7, dans lequel la fonction des abondances du premier groupe de biomarqueurs et des abondances du deuxième groupe de biomarqueurs est :

$$f(Ai, Aj) = b\left(\frac{\sum_{i\in13} Ai}{13} - \frac{\sum_{j\in2} Aj}{2}\right)$$

où *Ai est* une abondance de chaque biomarqueur *i* dans le premier groupe, *Aj* est une abondance de chaque biomarqueur *j* dans le deuxième groupe, $\sum_{i\in13} Ai$ est une somme des *Ai* sur tous les biomarqueurs *i* dans le premier groupe, $\sum_{j\in2} Aj$ est une somme des *Aj* sur tous les biomarqueurs *j* dans le deuxième groupe, et b est une constante, de préférence 10.

**9.** Procédé selon la revendication 1, dans lequel les biomarqueurs du premier groupe sont des genres bactériens choisis dans le groupe constitué de *Selenomonas*, *Lachnospiraceae, Peptococcus, Bacteroidaceae, Peptostreptococcus, Oribacterium, Veillonellaceae* et leurs combinaisons ; et le deuxième groupe de biomarqueurs est un genre bactérien d'*Abiotrophia*.

**10.** Procédé selon la revendication 9, dans lequel la condition cible est une sensibilité à la gingivite.

**11.** Procédé selon la revendication 10, dans lequel la fonction des abondances du premier groupe de biomarqueurs et des abondances du deuxième groupe de biomarqueurs est :

$$f(Ai, Aj) = b\left(\frac{\sum_{i\in7} Ai}{7} - \frac{\sum_{j\in1} Aj}{1}\right)$$

où *Ai est* une abondance de chaque biomarqueur *i* dans le premier groupe, *Aj* est une abondance de chaque biomarqueur *j* dans le deuxième groupe, $\sum_{i\in7} Ai$ est une somme des *Ai* sur tous les biomarqueurs i dans le premier groupe, $\sum_{j\in1} Aj$ est une somme des *Aj* sur tous les biomarqueurs *j* dans le deuxième groupe, et b est une constante, de préférence 10.

**12.** Procédé selon la revendication 1, dans lequel l'échantillon est choisi dans le groupe constitué d'un échantillon salivaire, un échantillon de plaque supragingivale, un échantillon de plaque subgingivale, un échantillon de plaque dentaire, et leurs combinaisons.

**13.** Procédé selon la revendication 1, dans lequel les abondances des premier et deuxième groupes de biomarqueurs sont mesurées par un procédé choisi dans le groupe constitué d'analyse d'ARNr 16S, de métagénomique, et d'une combinaison de celles-ci.

**14.** Système assisté par ordinateur pour l'exécution du procédé selon l'une quelconque des revendications 1 à 13.

Fig. 1A

| Time points | -21 (Day) | -14 | -7 | 0 | 7 | 14 | 21 | >21 (day) |
|---|---|---|---|---|---|---|---|---|
| Dental Prophylaxis | √ (BOP>2) | √ (BOP>2) | √ (BOP>2) | | | | | √ (BOP>2) |
| MGI record | √ | √ | √ | √ | √ | √ | √ | |
| Plaque collection | √ | | | √ | | | √ | |
| Oral Hygiene | From -21 to -1 day, twice monitored brushing and flossing per day | | | From 0 to 21 day, only twice mouth rinsing with water after meals per day | | | | Monitored oral hygiene |

Oral Hygiene Phase — Induced Gingivitis Phase — Recovery

NG*    Baseline    EG*

Fig. 1B

Fig. 2

Fig. 3A

Fig. 3B

Fig. 4A

Fig. 4B

Fig. 4C

Fig. 4D

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012100267 A1

### Non-patent literature cited in the description

- **BO LIU et al.** *Plos One,* June 2012, vol. 7 (6), e37919
- **RICARDO P. TELES et al.** *Journal of Clinical Periodontology,* 01 April 2010, vol. 37 (4), 313-123
- **MI ZHOU et al.** *Plos One,* 01 January 2013, vol. 8 (4), e 61516
- **T.F. MCNAMARA et al.** *Oral Surg Oral Med. Oral Pathol.,* 1972, vol. 34 (1), 41-8
- **J. TONZETICH.** *J. Periodontol.,* 1977, vol. 48 (1), 13-20
- **RAVEL J et al.** Vaginal microbiome of reproductive-age women. *Proc. Natl. Acad. Sci. U. S. A.,* 2011, vol. 108, 4680-4687
- **DRAY S ; DUFOUR AB.** The ade4 package: Implementing the duality diagram for ecologists. *Journal of Statistical Software,* 2007, vol. 22 (4), 1-20
- **EDGAR RC.** Search and clustering orders of magnitude faster than BLAST. *Bioinformatics,* 2010, vol. 26 (19), 2460-2461
- **GRIFFEN AL et al.** CORE: a phylogenetically-curated 16S rDNA database of the core oral microbiome. *PLoS One,* 2011, vol. 6 (4), e19051
- **HAMADY M ; LOZUPONE C ; KNIGHT R.** Fast UniFrac: facilitating high-throughput phylogenetic analyses of microbial communities including analysis of pyrosequencing and PhyloChip data. *ISME J,* 2010, vol. 4 (1), 17-27
- **SCHLOSS PD ; GEVERS D ; WESTCOTT SL.** Reducing the effects of PCR (Polymerase Chain Reaction) amplification and sequencing artifacts on 16S rRNA-based studies. *PLoS One,* 2011, vol. 6 (12), e27310